# EUROPEAN PATENT SPECIFICATION

(11) **EP 3 687 380 B1**
(45) Date of publication and mention of the grant of the patent: **11.11.2026**
(21) Application number: 18781990.9
(22) Date of filing: 24.09.2018
(51) Int. Cl.: A61B 5/00, A61B 5/145, A61B 5/1455, A61B 5/103, A61F 13/00, A61F 13/02, A61F 13/0203, A61M 1/00

(54) **PH SENSING FOR SENSOR ENABLED NEGATIVE PRESSURE WOUND MONITORING AND THERAPY APPARATUSES**
PH-FÜHLER FÜR SENSORAKTIVIERTE UNTERDRUCK-WUNDKONTROLL- UND THERAPIEGERÄTE
DÉTECTION DE PH POUR APPAREILS DE SURVEILLANCE ET DE THÉRAPIE DE PLAIE À PRESSION NÉGATIVE

(30) Priority: 27.09.2017 US 201762564126 P
(43) Date of publication of application: 05.08.2020
(73) Proprietor: Smith & Nephew plc, Watford, Hertfordshire WD18 8YE (GB)
(72) Inventor: COLE, Emma, Rhiannon, Hull HU3 2BN (GB); FRY, Nicholas, Hull HU3 2BN (GB); HUNT, Allan, Kenneth, Frazer, Grugeon, Hull HU3 2BN (GB); PHILLIPS, Marcus, Damian, Hull HU3 2BN (GB); STEWARD, Daniel, Lee, Hull HU3 2BN (GB)
(74) Representative: Appleyard Lees IP LLP
(86) International application number: PCT/EP2018/075802
(87) International publication number: WO 2019/063481

(56) References cited:
- WO-A1-2014/036577
- WO-A1-2017/041386
- DE-A1- 102012 211 015
- US-A1- 2016 262 672
- US-A1- 2017 000 407

## Description

### CROSS-REFERENCE TO RELATED APPLICATIONS

This application claims the benefit of U.S. Provisional Application No. 62/564,126, filed September 27, 2017 and entitled, PH SENSING FOR SENSOR ENABLED NEGATIVE PRESSURE WOUND MONITORING AND THERAPY APPARATUSES.

### FIELD

Embodiments described herein relate to apparatuses, systems, and methods for the monitoring or treatment of wounds, for example using dressings that sense pH of a wound.

### BACKGROUND

The treatment of open or chronic wounds that are too large to spontaneously close or otherwise fail to heal by means of applying negative pressure to the site of the wound is well known in the art. Negative pressure wound therapy (NPWT) systems currently known in the art commonly involve placing a cover that is impermeable or semi-permeable to fluids over the wound, using various means to seal the cover to the tissue of the patient surrounding the wound, and connecting a source of negative pressure (such as a vacuum pump) to the cover in a manner so that negative pressure is created and maintained under the cover. It is believed that such negative pressures promote wound healing by facilitating the formation of granulation tissue at the wound and assisting the body's normal inflammatory process while simultaneously removing excess fluid, which may contain adverse cytokines and/or bacteria. However, further improvements in NPWT are needed to fully realize the benefits of treatment.

Many different types of wound dressings are known for aiding in NPWT systems. These different types of wound dressings include many different types of materials and layers, for example, gauze, pads, foam pads or multi-layer wound dressings. Topical negative pressure therapy, sometimes referred to as vacuum assisted closure, negative pressure wound therapy, or reduced pressure wound therapy, is widely recognized as a beneficial mechanism for improving the healing rate of a wound. Such therapy is applicable to a broad range of wounds such as incisional wounds, open wounds and abdominal wounds or the like.

One example of a multi-layer wound dressing is the PICO dressing, available from Smith & Nephew, which includes a wound contact layer and a superabsorbent layer beneath a backing layer to provide a canister-less system for treating a wound with NPWT. The wound dressing may be sealed to a suction port providing connection to a length of tubing, which may be used to pump fluid out of the dressing and/or to transmit negative pressure from a pump to the wound dressing. Additionally, RENASYS-F, RENASYS-G, RENASYS-AB, and RENASYS-F/AB, available from Smith & Nephew, are additional examples of NPWT wound dressings and systems. Another example of a multi-layer wound dressing is the ALLEVYN Life dressing, available from Smith & Nephew, which includes a moist wound environment dressing that is used to treat the wound without the use of negative pressure.

The pH of a wound bed may provide valuable information regarding the state of wound healing. For example, researchers have found that prolonged chemical acidification of a wound bed may increase the healing rate in chronic wounds, speculating that such improved healing involves an increase in tissue oxygen availability through oxygen dissociation and reduced toxicity of bacterial end products, therefore stimulating wound healing. Chronic wound fluids generally contain elevated protease levels, which may have deleterious effects on wound healing, degrading *de novo* granulation tissue and endogenous, biologically active proteins such as growth factors and cytokines. The wound bed pH of chronic wounds tends to be alkaline or neutral (approximately pH of 7-8) when compared to intact surrounding skin (approximately pH of 5.5) but the pH of a chronic wound trends toward an acidic state during epithelialization. Protease activity is pH sensitive, peaking at about pH 7-8, but decreasing rapidly under more acidic conditions. When a wound is kept under more acidic condition, the fibroblasts proliferate more actively and the wound's healing process is stimulated to a greater extent than when in a neutral or alkaline condition.

However, prior art dressings for use in negative pressure wound therapy or other wound therapy provide little visualization or information of the condition of the wound beneath the dressing. This can require the dressing to be changed prematurely before the desired level of wound healing has occurred or, for absorbent dressings, prior to the full absorbent capacity of the dressing being reached to allow the clinician to inspect the healing and status of the wound. Some current dressings have limited and/or unsatisfactory methods or features of providing information of conditions of the wound, such as the pH. Therefore, improved monitoring of wound healing and/or wound pH is desirable.

Additionally, nearly all areas of medicine may benefit from improved information regarding the state of the tissue, organ, or system to be treated, particularly if such information is gathered in real-time during treatment. Many types of treatments are still routinely performed without the use of sensor data collection; instead, such treatments rely upon visual inspection by a caregiver or other limited means rather than quantitative sensor data. For example, in the case of wound treatment via dressings and/or negative pressure wound therapy, data collection is generally limited to visual inspection by a caregiver and often the underlying wounded tissue may be obscured by bandages or other visual impediments. Even intact, unwounded skin may have underlying damage that is not visible to the naked eye, such as a compromised vascular or deeper tissue damage that may lead to an ulcer. Similar to wound treatment, during orthopedic treatments requiring the immobilization of a limb with a cast or other encasement, only limited information is gathered on the underlying tissue. In instances of internal tissue repair, such as a bone plate, continued direct sensor-driven data collection is not performed. Further, braces and/or sleeves used to support musculoskeletal function do not monitor the functions of the underlying muscles or the movement of the limbs. Outside of direct treatments, common hospital room items such as beds and blankets could be improved by adding capability to monitor patient parameters. Documents WO2017041386A1, WO2014036577A1, DE102012211015A1 and US2017000407A1 relate to devices and systems for optical detection of a color of a pH-sensitive dye in a wound dressing.

Therefore, there is a need for improved sensor monitoring, particularly through the use of sensor-enabled substrates which can be incorporated into existing treatment regimes.

### SUMMARY

Some embodiments of the present disclosure relate to wound dressings. Some embodiments relate to wound monitoring systems. Some embodiments relate to methods of using wound dressings or wound monitoring systems. The invention is defined in the appended claims.

In some embodiments, a wound monitoring system includes a wound dressing and a controller. The wound dressing is configured to be positioned in contact with a wound and the wound dressing includes a plurality of sensors. The plurality of sensors is configured to measure a plurality of wound characteristics. The controller includes one or more processors. The controller is configured to be communicatively coupled to at least some of the plurality of sensors. In some embodiments, a kit may include any of the wound dressings described herein and/or any other component or feature described herein, for example a negative pressure source configured to be fluidically connected to the wound dressing.

In particular embodiments, a wound monitoring system comprises a wound dressing configured to be positioned in contact with a wound, the wound dressing comprising an optical sensor configured to measure a color; and a pH-sensitive material positioned on an underside of the wound dressing, the pH-sensitive material configured to change color in response to a change in a pH of the wound, wherein the optical sensor is further configured to detect the pH of the wound based on detection in the color change of the pH-sensitive material.

In some embodiments, the system may further comprise a non-pH-sensitive material positioned on the underside of the wound dressing, the non-pH-sensitive material configured to direct wound exudate to the pH-sensitive material. pH-sensitive material may comprise a gel or a foam. The non-pH-sensitive material may also comprise a gel or a foam and the gel or foam may be hydrophilic. The pH-sensitive material may be interspersed with pH-sensitive elements prior to formation of the gel or foam. The pH-sensitive elements may be dispersed heterogeneously. In embodiments, the pH-sensitive elements may be dispersed homogeneously. The non-pH-sensitive material may be configured to direct wound exudate to the pH-sensitive material. The non-pH material may be arranged as one or more channels on the underside of the wound dressing. The one or more channels may extend from the pH-sensitive material to an edge of the dressing. In certain embodiments, the system further comprises a controller, the controller configured to convert the color measured by the optical sensor to a pH value. The controller is configured to provide an indication of the pH value to a user. The controller may be further configured to display the pH value. In some embodiments, the pH-sensitive material may comprise adhesive material. In embodiments, the non-pH-sensitive material may comprise adhesive material. The pH-sensitive material may comprise a polyurethane.

In certain embodiments, a system such as described above may include a reference material, the reference material configured to maintain a stable color. The reference material may be incorporated into the wound dressing or be separate from the dressing.

In some embodiments, a method of monitoring the pH of a wound may comprise:
monitoring at least one of a wound or skin surrounding a wound with a wound dressing positioned in contact with the wound or skin surrounding the wound, the wound dressing comprising a pH-sensitive material configured to change color in response to a change in a pH of the wound and an optical sensor configured to detect a color change of the pH-sensitive material; and
computing with a processor a pH value based on the detected color change from the optical sensor.

In certain embodiments, the optical sensor may be configured to detect a color value of a reference material, and further normalizing a color value of the pH-sensitive material to the reference material. The reference material may be configured to maintain a stable color.

Any of the features, components, or details of any of the arrangements or embodiments disclosed in this application, including without limitation any of the pump embodiments and any of the negative pressure wound therapy embodiments disclosed below, are interchangeably combinable with any other features, components, or details of any of the arrangements or embodiments disclosed herein to form new arrangements and embodiments.

### BRIEF DESCRIPTION OF THE DRAWINGS

Figure 1A illustrates a negative pressure wound treatment system employing a flexible fluidic connector and a wound dressing capable of absorbing and storing wound exudate according to some embodiments;
Figure 1B illustrates a negative pressure wound treatment system employing a flexible fluidic connector and a wound dressing capable of absorbing and storing wound exudate according to some embodiments;
Figure 2A illustrates a negative pressure wound treatment system employing a flexible fluidic connector and a wound dressing capable of absorbing and storing wound exudate according to some embodiments;
Figure 2B illustrates a cross section of a fluidic connector connected to a wound dressing according to some embodiments;
Figure 2C illustrates of a negative pressure wound therapy system according to some embodiments;
Figure 2D illustrates a wound treatment system employing a wound dressing capable of absorbing and storing wound exudate to be used without negative pressure according to some embodiments;
Figure 3A illustrates a sensor array illustrating the sensor placement incorporated into a wound dressing component according to some embodiments;
Figure 3B illustrates a flexible sensor array including a sensor array portion, a tail portion, and a connector pad end portion according to some embodiments;
Figures 3C-3F show embodiments of the flexible circuit boards with four different sensor array geometries;
Figure 3G shows an embodiment of the sensor array portion of the sensor array design shown in Figure 3D in more detail;
Figure 3H illustrates a flexible sensor array incorporated into a perforated wound contact layer according to some embodiments;
Figure 3I illustrates a block diagram of a control module according to some embodiments;
Figure 4 illustrates an embodiment of a method for monitoring the pH in a wound;
Figure 5 depicts an embodiment of a wound therapy system employing a dressing including a pH-sensitive material beneath a plurality of sensors configured to detect changes in pH;
Figure 6 depicts a bottom side of an embodiment of a wound therapy system employing a wound dressing including channels for directing wound exudate to a sensor configured to detect changes in pH;
Figure 7 depicts an embodiment of a method for normalizing a sensor configured to detect changes in pH.

### DETAILED DESCRIPTION

Embodiments disclosed herein relate to apparatuses and methods of monitoring and treating biological tissue with sensor-enabled substrates. The embodiments disclosed herein are not limited to treatment or monitoring of a particular type of tissue or injury, instead the sensor-enabled technologies disclosed herein are broadly applicable to any type of therapy that may benefit from sensor-enabled substrates. Some implementations utilize sensors and data collection relied upon by health care providers to make both diagnostic and patient management decisions. International Patent Application No. PCT/IB2017/000693, filed May 5, 2017 titled "SENSOR ENABLED NEGATIVE PRESSURE WOUND THERAPY APPARATUS," published as WO 2017/195038 describes various example embodiments and features related to sensors, apparatuses, systems, and methods the treatment of wounds, for example using dressings in combination with negative pressure wound therapy.

Some embodiments disclosed herein relate to the use of sensors mounted on or embedded within substrates configured to be used in the treatment of both intact and damaged human or animal tissue. Such sensors may collect information about the surrounding tissue and transmit such information to a computing device or a caregiver to be utilized in further treatment. In certain embodiments, such sensors may be attached to the skin anywhere on the body, including areas for monitoring arthritis, temperature, or other areas that may be prone to problems and require monitoring. Sensors disclosed herein may also incorporate markers, such as radiopaque markers, to indicate the presence of the device, for example prior to performing an MRI or other technique.

The sensor embodiments disclosed herein may be used in combination with clothing. Non-limiting examples of clothing for use with embodiments of the sensors disclosed herein include shirts, pants, trousers, dresses, undergarments, outer-garments, gloves, shoes, hats, and other suitable garments. In certain embodiments, the sensor embodiments disclosed herein may be welded into or laminated into/onto the particular garments. The sensor embodiments may be printed directly onto the garment and/or embedded into the fabric. Breathable and printable materials such as microporous membranes may also be suitable.

Sensor embodiments disclosed herein may be incorporated into cushioning or bed padding, such as within a hospital bed, to monitor patient characteristics, such as any characteristic disclosed herein. In certain embodiments, a disposable film containing such sensors could be placed over the hospital bedding and removed/replaced as needed.

In some implementations, the sensor embodiments disclosed herein may incorporate energy harvesting, such that the sensor embodiments are self-sustaining. For example, energy may be harvested from thermal energy sources, kinetic energy sources, chemical gradients, or any suitable energy source.

The sensor embodiments disclosed herein may be utilized in rehabilitation devices and treatments, including sports medicine. For example, the sensor embodiments disclosed herein may be used in braces, sleeves, wraps, supports, and other suitable items. Similarly, the sensor embodiments disclosed herein may be incorporated into sporting equipment, such as helmets, sleeves, and/or pads. For example, such sensor embodiments may be incorporated into a protective helmet to monitor characteristics such as acceleration, which may be useful in concussion diagnosis.

The sensor embodiments disclosed herein may be used in coordination with surgical devices, for example, the NAVIO surgical system by Smith & Nephew Inc. In implementations, the sensor embodiments disclosed herein may be in communication with such surgical devices to guide placement of the surgical devices. In some implementations, the sensor embodiments disclosed herein may monitor blood flow to or away from the potential surgical site or ensure that there is no blood flow to a surgical site. Further surgical data may be collected to aid in the prevention of scarring and monitor areas away from the impacted area.

To further aid in surgical techniques, the sensors disclosed herein may be incorporated into a surgical drape to provide information regarding tissue under the drape that may not be immediately visible to the naked eye. For example, a sensor embedded flexible drape may have sensors positioned advantageously to provide improved area-focused data collection. In certain implementations, the sensor embodiments disclosed herein may be incorporated into the border or interior of a drape to create fencing to limit/ control the surgical theater.

Sensor embodiments as disclosed herein may also be utilized for pre-surgical assessment. For example, such sensor embodiments may be used to collect information about a potential surgical site, such as by monitoring skin and the underlying tissues for a possible incision site. For example, perfusion levels or other suitable characteristics may be monitored at the surface of the skin and deeper in the tissue to assess whether an individual patient may be at risk for surgical complications. Sensor embodiments such as those disclosed herein may be used to evaluate the presence of microbial infection and provide an indication for the use of antimicrobials. Further, sensor embodiments disclosed herein may collect further information in deeper tissue, such as identifying pressure ulcer damage and/or the fatty tissue levels.

The sensor embodiments disclosed herein may be utilized in cardiovascular monitoring. For example, such sensor embodiments may be incorporated into a flexible cardiovascular monitor that may be placed against the skin to monitor characteristics of the cardiovascular system and communicate such information to another device and/or a caregiver. For example, such a device may monitor pulse rate, oxygenation of the blood, and/or electrical activity of the heart. Similarly, the sensor embodiments disclosed herein may be utilized for neurophysiological applications, such as monitoring electrical activity of neurons.

The sensor embodiments disclosed herein may be incorporated into implantable devices, such as implantable orthopedic implants, including flexible implants. Such sensor embodiments may be configured to collect information regarding the implant site and transmit this information to an external source. In some embodiments, an internal source may also provide power for such an implant.

The sensor embodiments disclosed herein may also be utilized for monitoring biochemical activity on the surface of the skin or below the surface of the skin, such as lactose buildup in muscle or sweat production on the surface of the skin. In some embodiments, other characteristics may be monitored, such as glucose concentration, urine concentration, tissue pressure, skin temperature, skin surface conductivity, skin surface resistivity, skin hydration, skin maceration, and/or skin ripping.

Sensor embodiments as disclosed herein may be incorporated into Ear, Nose, and Throat (ENT) applications. For example, such sensor embodiments may be utilized to monitor recovery from ENT-related surgery, such as wound monitoring within the sinus passage.

As described in greater detail below, the sensor embodiments disclosed herein may encompass sensor printing technology with encapsulation, such as encapsulation with a polymer film. Such a film may be constructed using any polymer described herein, such as polyurethane. Encapsulation of the sensor embodiments may provide waterproofing of the electronics and protection from local tissue, local fluids, and other sources of potential damage.

In certain embodiments, the sensors disclosed herein may be incorporated into an organ protection layer such as disclosed below. Such a sensor-embedded organ protection layer may both protect the organ of interest and confirm that the organ protection layer is in position and providing protection. Further, a sensor-embedded organ protection layer may be utilized to monitor the underlying organ, such as by monitoring blood flow, oxygenation, and other suitable markers of organ health. In some embodiments, a sensor-enabled organ protection layer may be used to monitor a transplanted organ, such as by monitoring the fat and muscle content of the organ. Further, sensor-enabled organ protection layers may be used to monitor an organ during and after transplant, such as during rehabilitation of the organ.

The sensor embodiments disclosed herein may be incorporated into treatments for wounds (disclosed in greater detail below) or in a variety of other applications. Non-limiting examples of additional applications for the sensor embodiments disclosed herein include: monitoring and treatment of intact skin, cardiovascular applications such as monitoring blood flow, orthopedic applications such as monitoring limb movement and bone repair, neurophysiological applications such as monitoring electrical impulses, and any other tissue, organ, system, or condition that may benefit from improved sensor-enabled monitoring.

### Wound Therapy

Some embodiments disclosed herein relate to wound therapy for a human or animal body. Therefore, any reference to a wound herein can refer to a wound on a human or animal body, and any reference to a body herein can refer to a human or animal body. The disclosed technology embodiments may relate to preventing or minimizing damage to physiological tissue or living tissue, or to the treatment of damaged tissue (for example, a wound as described herein) wound with or without reduced pressure, including for example a source of negative pressure and wound dressing components and apparatuses. The apparatuses and components comprising the wound overlay and packing materials or internal layers, if any, are sometimes collectively referred to herein as dressings. In some embodiments, the wound dressing can be provided to be utilized without reduced pressure.

Some embodiments disclosed herein relate to wound therapy for a human or animal body. Therefore, any reference to a wound herein can refer to a wound on a human or animal body, and any reference to a body herein can refer to a human or animal body. The disclosed technology embodiments may relate to preventing or minimizing damage to physiological tissue or living tissue, or to the treatment of damaged tissue (for example, a wound as described herein).

As used herein the expression "wound" may include an injury to living tissue may be caused by a cut, blow, or other impact, typically one in which the skin is cut or broken. A wound may be a chronic or acute injury. Acute wounds occur as a result of surgery or trauma. They move through the stages of healing within a predicted timeframe. Chronic wounds typically begin as acute wounds. The acute wound can become a chronic wound when it does not follow the healing stages resulting in a lengthened recovery. It is believed that the transition from acute to chronic wound can be due to a patient being immuno-compromised.

Chronic wounds may include for example: venous ulcers (such as those that occur in the legs), which account for the majority of chronic wounds and mostly affect the elderly, diabetic ulcers (for example, foot or ankle ulcers), peripheral arterial disease, pressure ulcers, or epidermolysis bullosa (EB).

Examples of other wounds include, but are not limited to, abdominal wounds or other large or incisional wounds, either as a result of surgery, trauma, sterniotomies, fasciotomies, or other conditions, dehisced wounds, acute wounds, chronic wounds, subacute and dehisced wounds, traumatic wounds, flaps and skin grafts, lacerations, abrasions, contusions, burns, diabetic ulcers, pressure ulcers, stoma, surgical wounds, trauma and venous ulcers or the like.

Wounds may also include a deep tissue injury. Deep tissue injury is a term proposed by the National Pressure Ulcer Advisory Panel (NPUAP) to describe a unique form of pressure ulcers. These ulcers have been described by clinicians for many years with terms such as purple pressure ulcers, ulcers that are likely to deteriorate and bruises on bony prominences.

Wound may also include tissue at risk of becoming a wound as discussed herein. For example, tissue at risk may include tissue over a bony protuberance (at risk of deep tissue injury/insult) or pre-surgical tissue (for example, knee tissue) that may has the potential to be cut (for example, for joint replacement/surgical alteration/reconstruction).

Some embodiments relate to methods of treating a wound with the technology disclosed herein in conjunction with one or more of the following: advanced footwear, turning a patient, offloading (such as, offloading diabetic foot ulcers), treatment of infection, systemix, antimicrobial, antibiotics, surgery, removal of tissue, affecting blood flow, physiotherapy, exercise, bathing, nutrition, hydration, nerve stimulation, ultrasound, electrostimulation, oxygen therapy, microwave therapy, active agents ozone, antibiotics, antimicrobials, or the like.

Alternatively or additionally, a wound may be treated using topical negative pressure and/or traditional advanced wound care, which is not aided by the using of applied negative pressure (may also be referred to as non-negative pressure therapy).

Advanced wound care may include use of an absorbent dressing, an occlusive dressing, use of an antimicrobial and/or debriding agents in a wound dressing or adjunct, a pad (for example, a cushioning or compressive therapy, such as stockings or bandages), or the like.

In some embodiments, treatment of such wounds can be performed using traditional wound care, wherein a dressing can be applied to the wound to facilitate and promote healing of the wound. Some embodiments relate to methods of manufacturing a wound dressing comprising providing a wound dressing as disclosed herein. The wound dressings that may be utilized in conjunction with the disclosed technology include any known dressing in the art. The technology is applicable to negative pressure therapy treatment as well as non-negative pressure therapy treatment. In some embodiments, a wound dressing comprises one or more absorbent layer(s). The absorbent layer may be a foam or a superabsorbent.

In some embodiments, wound dressings may comprise a dressing layer including a polysaccharide or modified polysaccharide, a polyvinylpyrrolidone, a polyvinyl alcohol, a polyvinyl ether, a polyurethane, a polyacrylate, a polyacrylamide, collagen, or gelatin or mixtures thereof. Dressing layers comprising the polymers listed are known in the art as being useful for forming a wound dressing layer for either negative pressure therapy or non-negative pressure therapy.

In some embodiments, the polymer matrix may be a polysaccharide or modified polysaccharide. In some embodiments, the polymer matrix may be a cellulose. Cellulose material may include hydrophilically modified cellulose such as methyl cellulose, carboxymethyl cellulose (CMC), carboxymethyl cellulose (CEC), ethyl cellulose, propyl cellulose, hydroxyethyl cellulose, hydroxypropyl cellulose, hydroxypropylmethyl cellulose, carboxyethyl sulphonate cellulose, cellulose alkyl sulphonate, or mixtures thereof.

In certain embodiments, cellulose material may be cellulose alkyl sulphonate. The alkyl moiety of the alkyl sulphonate substituent group may have an alkyl group having 1 to 6 carbon atoms, such as methyl, ethyl, propyl, or butyl. The alkyl moiety may be branched or unbranched, and hence suitable propyl sulphonate substituents may be 1- or 2-methyl-ethylsulphonate. Butyl sulphonate substituents may be 2-ethyl-ethylsulphonate, 2,2-dimethyl-ethylsulphonate, or 1,2-dimethyl-ethylsulphonate. The alkyl sulphonate substituent group may be ethyl sulphonate. The cellulose alkyl sulphonate is described in WO10061225, US2016/114074, US2006/0142560, or US 5,703,225.

Cellulose alkyl sulfonates may have varying degrees of substitution, the chain length of the cellulose backbone structure, and the structure of the alkyl sulfonate substituent. Solubility and absorbency are largely dependent on the degree of substitution: as the degree of substitution is increased, the cellulose alkyl sulfonate becomes increasingly soluble. It follows that, as solubility increases, absorbency increases.

In some embodiments, a wound dressing also comprises a top or cover layer. The thickness of the wound dressing disclosed herein may be between 1 to 20, or 2 to 10, or 3 to 7 mm. In some embodiments, the disclosed technology may be used in conjunction with a non-negative pressure dressing. A non-negative pressure wound dressing suitable for providing protection at a wound site may comprise: an absorbent layer for absorbing wound exudate and an obscuring element for at least partially obscuring a view of wound exudate absorbed by the absorbent layer in use.

The obscuring element may be partially translucent. The obscuring element may be a masking layer. The non-negative pressure wound dressing may further comprise a region in or adjacent the obscuring element for allowing viewing of the absorbent layer. For example, the obscuring element layer may be provided over a central region of the absorbent layer and not over a border region of the absorbent layer. In some embodiments, the obscuring element is of hydrophilic material or is coated with a hydrophilic material.

The obscuring element may comprise a three-dimensional knitted spacer fabric. The spacer fabric is known in the art and may include a knitted spacer fabric layer. The obscuring element may further comprise an indicator for indicating the need to change the dressing. In some embodiments, the obscuring element is provided as a layer at least partially over the absorbent layer, further from a wound site than the absorbent layer in use.

The non-negative pressure wound dressing may further comprise a plurality of openings in the obscuring element for allowing fluid to move therethrough. The obscuring element may comprise, or may be coated with, a material having size-exclusion properties for selectively permitting or preventing passage of molecules of a predetermined size or weight.

The obscuring element may be configured to at least partially mask light radiation having wavelength of 600 nm and less. The obscuring element may be configured to reduce light absorption by 50% or more. The obscuring element may be configured to yield a CIE L* value of 50 or more, and optionally 70 or more. In some embodiments, the obscuring element may be configured to yield a CIE L* value of 70 or more. In some embodiments, the non-negative pressure wound dressing may further comprise at least one of a wound contact layer, a foam layer, an odor control element, a pressure-resistant layer and a cover layer.

In some embodiments, the cover layer is present, and the cover layer is a translucent film. Typically, the translucent film has a moisture vapour permeability of 500g/m2/24hours or more. The translucent film may be a bacterial barrier. In some embodiments, the non-negative pressure wound dressing as disclosed herein comprises the wound contact layer and the absorbent layer overlies the wound contact layer. The wound contact layer carries an adhesive portion for forming a substantially fluid tight seal over the wound site. The non-negative pressure wound dressing as disclosed herein may comprise the obscuring element and the absorbent layer being provided as a single layer.

**In** some embodiments, the non-negative pressure wound dressing disclosed herein comprises the foam layer, and the obscuring element is of a material comprising components that may be displaced or broken by movement of the obscuring element.

**In** some embodiments, the non-negative pressure wound dressing comprises an odor control element, and in another embodiment the dressing does not include an odor control element. When present, the odor control element may be dispersed within or adjacent the absorbent layer or the obscuring element. Alternatively, when present the odor control element may be provided as a layer sandwiched between the foam layer and the absorbent layer.

**In** some embodiments, the disclosed technology for a non-negative pressure wound dressing comprises a method of manufacturing a wound dressing, comprising: providing an absorbent layer for absorbing wound exudate; and providing an obscuring element for at least partially obscuring a view of wound exudate absorbed by the absorbent layer in use.

**In** some embodiments, the non-negative pressure wound dressing may be suitable for providing protection at a wound site, comprising: an absorbent layer for absorbing wound exudate; and a shielding layer provided over the absorbent layer, and further from a wound-facing side of the wound dressing than the absorbent layer. The shielding layer may be provided directly over the absorbent layer. In some embodiments, the shielding layer comprises a three-dimensional spacer fabric layer.

The shielding layer increases the area over which a pressure applied to the dressing is transferred by 25% or more or the initial area of application. For example the shielding layer increases the area over which a pressure applied to the dressing is transferred by 50% or more, and optionally by 100% or more, and optionally by 200% or more. The shielding layer may comprise 2 or more sub-layers, wherein a first sub-layer comprises through holes and a further sub-layer comprises through holes and the through holes of the first sub-layer are offset from the through holes of the further sub-layer.

The non-negative pressure wound dressing as disclosed herein may further comprise a permeable cover layer for allowing the transmission of gas and vapour therethrough, the cover layer provided over the shielding layer, wherein through holes of the cover layer are offset from through holes of the shielding layer. The non-negative pressure wound dressing may be suitable for treatment of pressure ulcers.

A more detailed description of the non-negative pressure dressing disclosed hereinabove is provided in WO2013007973. In some embodiments, the non-negative pressure wound dressing may be a multi-layered wound dressing comprising: a fibrous absorbent layer for absorbing exudate from a wound site; and a support layer configured to reduce shrinkage of at least a portion of the wound dressing.

In some embodiments, the multi-layered wound dressing disclosed herein, further comprises a liquid impermeable film layer, wherein the support layer is located between the absorbent layer and the film layer. The support layer disclosed herein may comprise a net. The net may comprise a geometric structure having a plurality of substantially geometric apertures extending therethrough. The geometric structure may for example comprise a plurality of bosses substantially evenly spaced and joined by polymer strands to form the substantially geometric apertures between the polymer strands.

The net may be formed from high density polyethylene. The apertures may have an area from 0.005 to 0.32 mm2. The support layer may have a tensile strength from 0.05 to 0.06 Nm. The support layer may have a thickness of from 50 to 150 µm.

In some embodiments, the support layer is located directly adjacent the absorbent layer. Typically, the support layer is bonded to fibers in a top surface of the absorbent layer. The support layer may further comprise a bonding layer, wherein the support layer is heat laminated to the fibers in the absorbent layer via the bonding layer. The bonding layer may comprise a low melting point adhesive such as ethylene-vinyl acetate adhesive.

In some embodiments, the multi-layered wound dressing disclosed herein further comprises an adhesive layer attaching the film layer to the support layer. In some embodiments, the multi-layered wound dressing disclosed herein further comprises a wound contact layer located adjacent the absorbent layer for positioning adjacent a wound. The multi-layered wound dressing may further comprise a fluid transport layer between the wound contact layer and the absorbent layer for transporting exudate away from a wound into the absorbent layer.

A more detailed description of the multi-layered wound dressing disclosed hereinabove is provided in GB patent application filed on 28 October 2016 with application number GB1618298.2, the disclosed technology may be incorporated in a wound dressing comprising a vertically lapped material comprising: a first layer of an absorbing layer of material, and a second layer of material, wherein the first layer being constructed from at least one layer of non-woven textile fibers, the non-woven textile fibers being folded into a plurality of folds to form a pleated structure. In some embodiments, the wound dressing further comprises a second layer of material that is temporarily or permanently connected to the first layer of material.

Typically the vertically lapped material has been slitted. In some embodiments, the first layer has a pleated structure having a depth determined by the depth of pleats or by the slitting width. The first layer of material may be a moldable, lightweight, fiber-based material, blend of material or composition layer.

The first layer of material may comprise one or more of manufactured fibers from synthetic, natural or inorganic polymers, natural fibers of a cellulosic, proteinaceous or mineral source. The wound dressing may comprise two or more layers of the absorbing layer of material vertically lapped material stacked one on top of the other, wherein the two or more layers have the same or different densities or composition. The wound dressing may in some embodiments comprise only one layer of the absorbing layer of material vertically lapped material.

The absorbing layer of material is a blend of natural or synthetic, organic or inorganic fibers, and binder fibers, or bicomponent fibers typically PET with a low melt temperature PET coating to soften at specified temperatures and to act as a bonding agent in the overall blend. In some embodiments, the absorbing layer of material may be a blend of 5 to 95 % thermoplastic polymer, and 5 to 95 wt % of a cellulose or derivative thereof. In some embodiments, the wound dressing disclosed herein has a second layer comprises a foam or a dressing fixative. The foam may be a polyurethane foam. The polyurethane foam may have an open or closed pore structure.

The dressing fixative may include bandages, tape, gauze, or backing layer. In some embodiments, the wound dressing as disclosed herein comprises the absorbing layer of material connected directly to a second layer by lamination or by an adhesive, and the second layer is connected to a dressing fixative layer. The adhesive may be an acrylic adhesive, or a silicone adhesive.

In some embodiments, the wound dressing as disclosed herein further comprises layer of a superabsorbent fiber, or a viscose fiber or a polyester fiber. In some embodiments, the wound dressing as disclosed herein further comprises a backing layer. The backing layer may be a transparent or opaque film. Typically the backing layer comprises a polyurethane film (typically a transparent polyurethane film). A more detailed description of the multi-layered wound dressing disclosed hereinabove is provided in GB patent applications filed on 12 December 2016 with application number GB1621057.7; and 22 June 2017 with application number GB17099870.

In some embodiments, the non-negative pressure wound dressing may comprise an absorbent component for a wound dressing, the component comprising a wound contacting layer comprising gel forming fibers bound to a foam layer, wherein the foam layer is bound directly to the wound contact layer by an adhesive, polymer based melt layer, by flame lamination or by ultrasound. The absorbent component may be in a sheet form. The wound contacting layer may comprise a layer of woven or non-woven or knitted gel forming fibers.

The foam layer may be an open cell foam, or closed cell foam, typically an open cell foam. The foam layer is a hydrophilic foam. The wound dressing may comprise the component that forms an island in direct contact with the wound surrounded by periphery of adhesive that adheres the dressing to the wound. The adhesive may be a silicone or acrylic adhesive, typically a silicone adhesive. The wound dressing may be covered by a film layer on the surface of the dressing furthest from the wound.

A more detailed description of the wound dressing of this type hereinabove is provided in EP2498829. In some embodiments, the non-negative pressure wound dressing may comprise a multi layered wound dressing for use on wounds producing high levels of exudate, characterized in that the dressing comprising: a transmission layer having an MVTR of at least 300 gm2/24 hours, an absorbent core comprising gel forming fibers capable of absorbing and retaining exudate, a wound contacting layer comprising gel forming fibers which transmits exudate to the absorbent core and a keying layer positioned on the absorbent core, the absorbent core and wound contacting layer limiting the lateral spread of exudate in the dressing to the region of the wound.

The wound dressing may be capable of handling at least 6g (or 8g and 15g) of fluid per 10cm2 of dressing in 24 hours. The wound dressing may comprise gel forming fibers that are chemically modified cellulosic fibers in the form of a fabric. The fibers may include carboxymethylated cellulose fibers, typically sodium carboxymethylcellulose fiber. The wound dressing may comprise a wound contact layer with a lateral wicking rate from 5mm per minute to 40mm per minute. The wound contact layer may have a fiber density between 25gm2 and 55gm2, such as 35gm2. The absorbent core may have an absorbency of exudate of at least 10g/g, and typically a rate of lateral wicking of less the 20mm per minute. The absorbent core may have a blend in the range of up to 25% cellulosic fibers by weight and 75% to 100% gel forming fibers by weight.

Alternatively, the absorbent core may have a blend in the range of up to 50% cellulosic fibers by weight and 50% to 100% gel forming fibers by weight. For example the blend is in the range of 50% cellulosic fibers by weight and 50% gel forming fibers by weight. The fiber density in the absorbent core may be between 150gm2 and 250gm2, or about 200 gm2. The wound dressing when wet may have shrinkage that is less than 25 % or less than 15 % of its original size/dimension. The wound dressing may comprise a transmission layer and the layer is a foam. The transmission layer may be a polyurethane foam laminated to a polyurethane film.

The wound dressing may comprise one or more layers selected from the group comprising a soluble medicated film layer; an odor-absorbing layer; a spreading layer and an additional adhesive layer. The wound dressing may be 2mm and 4mm thick. The wound dressing may be characterized in that the keying layer bonds the absorbent core to a neighboring layer. In some embodiments, the keying layer may be positioned on either the wound facing side of the absorbent core or the non-wound facing side of the absorbent core. In some embodiments, the keying layer is positioned between the absorbent core and the wound contact layer. The keying layer is a polyamide web.

A more detailed description of the wound dressing of this type hereinabove is provided in EP1718257. In some embodiments, the non-negative pressure wound dressing may be a compression bandage. Compression bandages are known for use in the treatment of oedema and other venous and lymphatic disorders, e.g., of the lower limbs. A compression bandage systems typically employ multiple layers including a padding layer between the skin and the compression layer or layers. The compression bandage may be useful for wounds such as handling venous leg ulcers.

The compression bandage in some embodiments may comprise a bandage system comprising an inner skin facing layer and an elastic outer layer, the inner layer comprising a first ply of foam and a second ply of an absorbent nonwoven web, the inner layer and outer layer being sufficiently elongated so as to be capable of being wound about a patient's limb. A compression bandage of this type is disclosed in WO99/58090.

In some embodiments, the compression bandage system comprises: a) an inner skin facing, elongated, elastic bandage comprising: (i) an elongated, elastic substrate, and (ii) an elongated layer of foam, said foam layer being affixed to a face of said substrate and extending 33% or more across said face of substrate in transverse direction and 67% or more across said face of substrate in longitudinal direction; and b) an outer, elongated, self-adhering elastic bandage; said bandage having a compressive force when extended; wherein, in use, said foam layer of the inner bandage faces the skin and the outer bandage overlies the inner bandage. A compression bandage of this type is disclosed in WO2006/110527. In some embodiments other compression bandage systems such as those disclosed in US 6,759,566 and US 2002/0099318.

### Negative Pressure Wound Dressing

In some embodiments, treatment of such wounds can be performed using negative pressure wound therapy, wherein a reduced or negative pressure can be applied to the wound to facilitate and promote healing of the wound. It will also be appreciated that the wound dressing and methods as disclosed herein may be applied to other parts of the body, and are not necessarily limited to treatment of wounds.

It will be understood that embodiments of the present disclosure are generally applicable to use in topical negative pressure ("TNP") therapy systems. Briefly, negative pressure wound therapy assists in the closure and healing of many forms of "hard to heal" wounds by reducing tissue oedema; encouraging blood flow and granular tissue formation; removing excess exudate and may reduce bacterial load (and thus infection risk). In addition, the therapy allows for less disturbance of a wound leading to more rapid healing. TNP therapy systems may also assist on the healing of surgically closed wounds by removing fluid and by helping to stabilize the tissue in the apposed position of closure. A further beneficial use of TNP therapy can be found in grafts and flaps where removal of excess fluid is important and close proximity of the graft to tissue is required in order to ensure tissue viability.

Negative pressure therapy can be used for the treatment of open or chronic wounds that are too large to spontaneously close or otherwise fail to heal by means of applying negative pressure to the site of the wound. Topical negative pressure (TNP) therapy or negative pressure wound therapy (NPWT) involves placing a cover that is impermeable or semi-permeable to fluids over the wound, using various means to seal the cover to the tissue of the patient surrounding the wound, and connecting a source of negative pressure (such as a vacuum pump) to the cover in a manner so that negative pressure is created and maintained under the cover. It is believed that such negative pressures promote wound healing by facilitating the formation of granulation tissue at the wound site and assisting the body's normal inflammatory process while simultaneously removing excess fluid, which may contain adverse cytokines or bacteria.

Some of the dressings used in NPWT can include many different types of materials and layers, for example, gauze, pads, foam pads or multi-layer wound dressings. One example of a multi-layer wound dressing is the PICO dressing, available from Smith & Nephew, includes a wound contact layer and a superabsorbent layer beneath a backing layer to provide a canister-less system for treating a wound with NPWT. The wound dressing may be sealed to a suction port providing connection to a length of tubing, which may be used to pump fluid out of the dressing or to transmit negative pressure from a pump to the wound dressing. Additionally, RENASYS-F, RENASYS-G, RENASYS-AB, and RENASYS-F/AB, available from Smith & Nephew, are additional examples of NPWT wound dressings and systems. Another example of a multi-layer wound dressing is the ALLEVYN Life dressing, available from Smith & Nephew, which includes a moist wound environment dressing that is used to treat the wound without the use of negative pressure.

As is used herein, reduced or negative pressure levels, such as -X mmHg, represent pressure levels relative to normal ambient atmospheric pressure, which can correspond to 760 mmHg (or 1 atm, 29.93 inHg, 101.325 kPa, 14.696 psi, etc.). Accordingly, a negative pressure value of -X mmHg reflects absolute pressure that is X mmHg below 760 mmHg or, in other words, an absolute pressure of (760-X) mmHg. In addition, negative pressure that is "less" or "smaller" than X mmHg corresponds to pressure that is closer to atmospheric pressure (such as,-40 mmHg is less than -60 mmHg). Negative pressure that is "more" or "greater" than -X mmHg corresponds to pressure that is further from atmospheric pressure (such as, -80 mmHg is more than -60 mmHg). In some embodiments, local ambient atmospheric pressure is used as a reference point, and such local atmospheric pressure may not necessarily be, for example, 760 mmHg.

The negative pressure range for some embodiments of the present disclosure can be approximately -80 mmHg, or between about -20 mmHg and -200 mmHg. Note that these pressures are relative to normal ambient atmospheric pressure, which can be 760 mmHg. Thus, -200 mmHg would be about 560 mmHg in practical terms. In some embodiments, the pressure range can be between about -40 mmHg and -150 mmHg. Alternatively a pressure range of up to -75 mmHg, up to -80 mmHg or over -80 mmHg can be used. Also in other embodiments a pressure range of below -75 mmHg can be used. Alternatively, a pressure range of over approximately -100 mmHg, or even -150 mmHg, can be supplied by the negative pressure apparatus.

In some embodiments of wound closure devices described herein, increased wound contraction can lead to increased tissue expansion in the surrounding wound tissue. This effect may be increased by varying the force applied to the tissue, for example by varying the negative pressure applied to the wound over time, possibly in conjunction with increased tensile forces applied to the wound via embodiments of the wound closure devices. In some embodiments, negative pressure may be varied over time for example using a sinusoidal wave, square wave, or in synchronization with one or more patient physiological indices (such as, heartbeat). Examples of such applications where additional disclosure relating to the preceding may be found include U.S. Patent No. 8,235,955, titled "Wound treatment apparatus and method," issued on August 7, 2012; and U.S. Patent No. 7,753,894, titled "Wound cleansing apparatus with stress," issued July 13, 2010.

Embodiments of the wound dressings, wound dressing components, wound treatment apparatuses and methods described herein may also be used in combination or in addition to those described in International Application No. PCT/IB2013/001469, filed May 22, 2013, published as WO 2013/175306 A2 on November 28, 2013, titled "APPARATUSES AND METHODS FOR NEGATIVE PRESSURE WOUND THERAPY," U.S. Patent Application No. 14/418,908, filed January 30, 2015, published as US 2015/0190286 A1 on July 9, 2015, titled "WOUND DRESSING AND METHOD OF TREATMENT,". Embodiments of the wound dressings, wound dressing components, wound treatment apparatuses and methods described herein may also be used in combination or in addition to those described in U.S. Patent Application No. 13/092,042, filed April 21, 2011, published as US2011/0282309, titled "WOUND DRESSING AND METHOD OF USE," and U.S. Patent Application No. 14/715,527, filed May 18, 2015, published as US2016/0339158 A1 on November 24, 2016, titled "FLUIDIC CONNECTOR FOR NEGATIVE PRESSURE WOUND THERAPY," including further details relating to embodiments of wound dressings, the wound dressing components and principles, and the materials used for the wound dressings.

Additionally, some embodiments related to TNP wound treatment comprising a wound dressing in combination with a pump or associated electronics described herein may also be used in combination or in addition to those described in International Application PCT/EP2016/059329 filed April 26, 2016, published as WO 2016/174048 on November 3, 2016, entitled "REDUCED PRESSURE APPARATUS AND METHODS,".

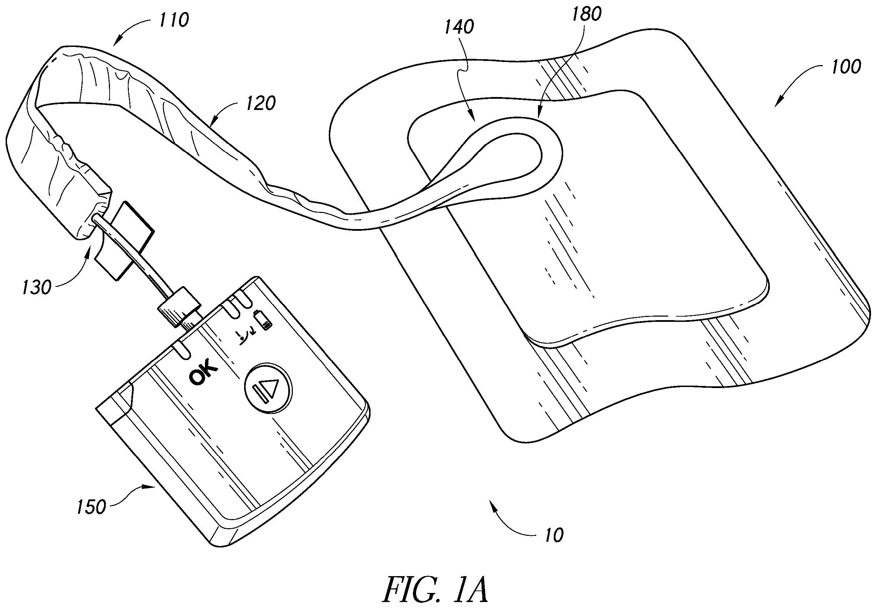

Figures 1A-B illustrate embodiments of a negative pressure wound treatment system 10 employing a wound dressing 100 in conjunction with a fluidic connector 110. Here, the fluidic connector 110 may comprise an elongate conduit, for example, a bridge 120 having a proximal end 130 and a distal end 140, and an applicator 180 at the distal end 140 of the bridge 120. An optional coupling 160 can be disposed at the proximal end 130 of the bridge 120. A cap 170 may be provided with the system (and can in some cases, as illustrated, be attached to the coupling 160). The cap 170 can be useful in preventing fluids from leaking out of the proximal end 130. The system 10 may include a source of negative pressure such as a pump or negative pressure unit 150 capable of supplying negative pressure. The pump may comprise a canister or other container for the storage of wound exudates and other fluids that may be removed from the wound. A canister or container may also be provided separate from the pump. In some embodiments, such as illustrated in Figures 1A-1B, the pump 150 can be a canisterless pump such as the PICO^{™} pump, as sold by Smith & Nephew. The pump 150 may be connected to the coupling 160 via a tube 190, or the pump 150 may be connected directly to the coupling 160 or directly to the bridge 120. In use, the dressing 100 is placed over a suitably-prepared wound, which may in some cases be filled with a wound packing material such as foam or gauze. The applicator 180 of the fluidic connector 110 has a sealing surface that is placed over an aperture in the dressing 100 and is sealed to the top surface of the dressing 100. Either before, during, or after connection of the fluidic connector 110 to the dressing 100, the pump 150 is connected via the tube 190 to the coupling 160, or is connected directly to the coupling 160 or to the bridge 120. The pump is then activated, thereby supplying negative pressure to the wound. Application of negative pressure may be applied until a desired level of healing of the wound is achieved.

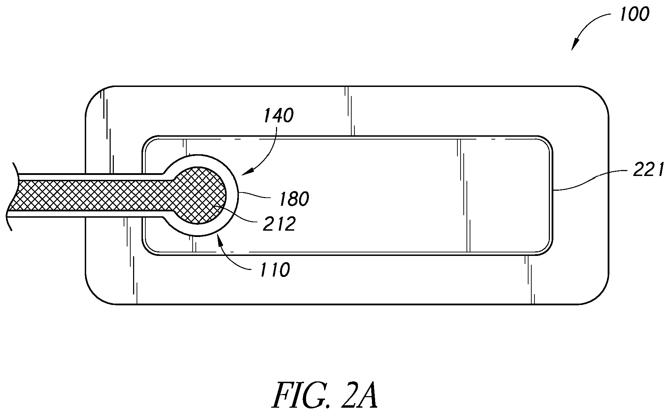

As shown in Figure 2A, the fluidic connector 110 preferably comprises an enlarged distal end, or head 140 that is in fluidic communication with the dressing 100 as will be described in further detail below. In one embodiment, the enlarged distal end has a round or circular shape. The head 140 is illustrated here as being positioned near an edge of the dressing 100, but may also be positioned at any location on the dressing. For example, some embodiments may provide for a centrally or off-centered location not on or near an edge or corner of the dressing 100. In some embodiments, the dressing 10 may comprise two or more fluidic connectors 110, each comprising one or more heads 140, in fluidic communication therewith. In a preferred embodiment, the head 140 may measure 30mm along its widest edge. The head 140 forms at least in part the applicator 180, described above, that is configured to seal against a top surface of the wound dressing.

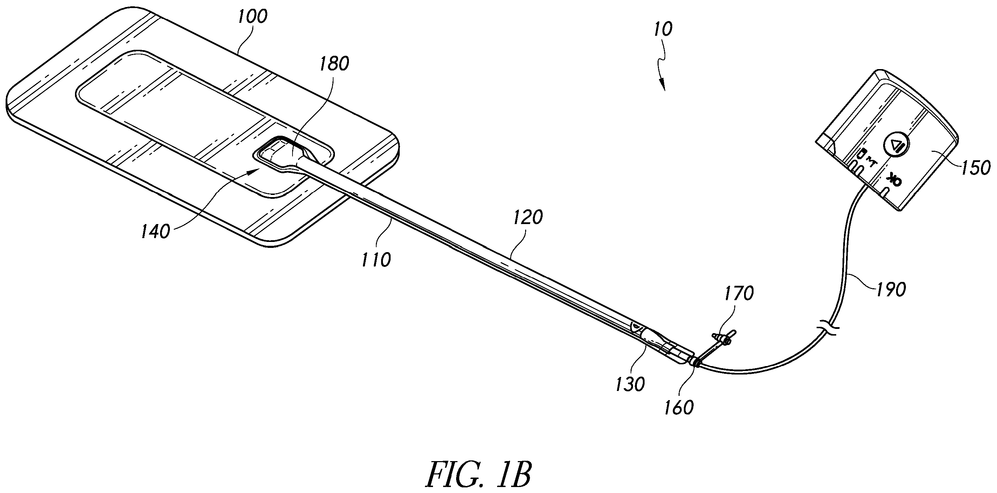

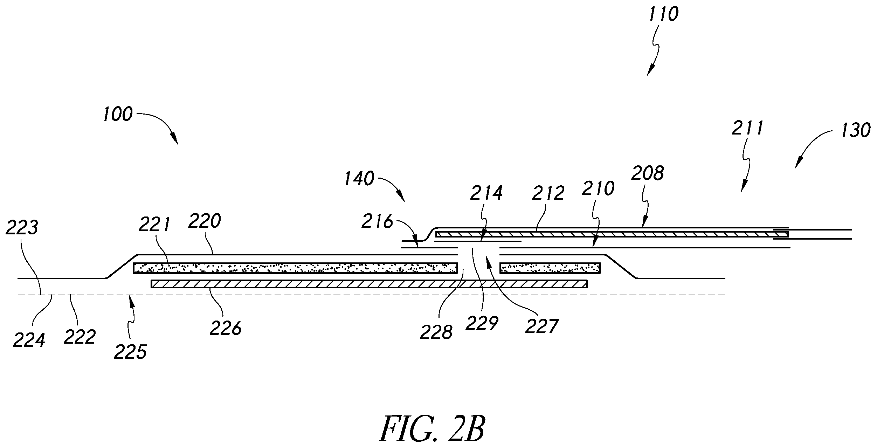

Figure 2B illustrates a cross-section through a wound dressing 100 similar to the wound dressing 10 as shown in Figure 1B and described in International Patent Publication WO2013175306 A2, along with fluidic connector 110. The wound dressing 100, which can alternatively be any wound dressing embodiment disclosed herein or any combination of features of any number of wound dressing embodiments disclosed herein, can be located in or over a wound to be treated. The dressing 100 may be placed as to form a sealed cavity over the wound. In a preferred embodiment, the dressing 100 comprises a top or cover layer, or backing layer 220 attached to an optional wound contact layer 222, both of which are described in greater detail below. These two layers 220, 222 are preferably joined or sealed together so as to define an interior space or chamber. This interior space or chamber may comprise additional structures that may be adapted to distribute or transmit negative pressure, store wound exudate and other fluids removed from the wound, and other functions which will be explained in greater detail below. Examples of such structures, described below, include a transmission layer 226 and an absorbent layer 221.

As used herein the upper layer, top layer, or layer above refers to a layer furthest from the surface of the skin or wound while the dressing is in use and positioned over the wound. Accordingly, the lower surface, lower layer, bottom layer, or layer below refers to the layer that is closest to the surface of the skin or wound while the dressing is in use and positioned over the wound.

As illustrated in Figure 2B, the wound contact layer 222 can be a polyurethane layer or polyethylene layer or other flexible layer which is perforated, for example via a hot pin process, laser ablation process, ultrasound process or in some other way or otherwise made permeable to liquid and gas. The wound contact layer 222 has a lower surface 224 and an upper surface 223. The perforations 225 preferably comprise through holes in the wound contact layer 222 which enable fluid to flow through the layer 222. The wound contact layer 222 helps prevent tissue ingrowth into the other material of the wound dressing. Preferably, the perforations are small enough to meet this requirement while still allowing fluid to flow therethrough. For example, perforations formed as slits or holes having a size ranging from 0.025 mm to 1.2 mm are considered small enough to help prevent tissue ingrowth into the wound dressing while allowing wound exudate to flow into the dressing. In some configurations, the wound contact layer 222 may help maintain the integrity of the entire dressing 100 while also creating an air tight seal around the absorbent pad in order to maintain negative pressure at the wound.

Some embodiments of the wound contact layer 222 may also act as a carrier for an optional lower and upper adhesive layer (not shown). For example, a lower pressure sensitive adhesive may be provided on the lower surface 224 of the wound dressing 100 whilst an upper pressure sensitive adhesive layer may be provided on the upper surface 223 of the wound contact layer. The pressure sensitive adhesive, which may be a silicone, hot melt, hydrocolloid or acrylic based adhesive or other such adhesives, may be formed on both sides or optionally on a selected one or none of the sides of the wound contact layer. When a lower pressure sensitive adhesive layer is utilized may be helpful to adhere the wound dressing 100 to the skin around a wound. In some embodiments, the wound contact layer may comprise perforated polyurethane film. The lower surface of the film may be provided with a silicone pressure sensitive adhesive and the upper surface may be provided with an acrylic pressure sensitive adhesive, which may help the dressing maintain its integrity. In some embodiments, a polyurethane film layer may be provided with an adhesive layer on both its upper surface and lower surface, and all three layers may be perforated together.

A layer 226 of porous material can be located above the wound contact layer 222. This porous layer, or transmission layer, 226 allows transmission of fluid including liquid and gas away from a wound into upper layers of the wound dressing. In particular, the transmission layer 226 preferably ensures that an open air channel can be maintained to communicate negative pressure over the wound area even when the absorbent layer has absorbed substantial amounts of exudates. The layer 226 should preferably remain open under the typical pressures that will be applied during negative pressure wound therapy as described above, so that the whole wound sees an equalized negative pressure. The layer 226 may be formed of a material having a three dimensional structure. For example, a knitted or woven spacer fabric (for example Baltex 7970 weft knitted polyester) or a non-woven fabric could be used.

In some embodiments, the transmission layer 226 comprises a 3D polyester spacer fabric layer including a top layer (that is to say, a layer distal from the wound-bed in use) which is a 84/144 textured polyester, and a bottom layer (that is to say, a layer which lies proximate to the wound bed in use) which is a 10 denier flat polyester and a third layer formed sandwiched between these two layers which is a region defined by a knitted polyester viscose, cellulose or the like monofilament fiber. Other materials and other linear mass densities of fiber could of course be used.

Whilst reference is made throughout this disclosure to a monofilament fiber it will be appreciated that a multistrand alternative could of course be utilized. The top spacer fabric thus has more filaments in a yarn used to form it than the number of filaments making up the yarn used to form the bottom spacer fabric layer.

This differential between filament counts in the spaced apart layers helps control moisture flow across the transmission layer. Particularly, by having a filament count greater in the top layer, that is to say, the top layer is made from a yarn having more filaments than the yarn used in the bottom layer, liquid tends to be wicked along the top layer more than the bottom layer. In use, this differential tends to draw liquid away from the wound bed and into a central region of the dressing where the absorbent layer 221 helps lock the liquid away or itself wicks the liquid onwards towards the cover layer where it can be transpired.

Preferably, to improve the liquid flow across the transmission layer 226 (that is to say perpendicular to the channel region formed between the top and bottom spacer layers, the 3D fabric may be treated with a dry cleaning agent (such as, but not limited to, Perchloro Ethylene) to help remove any manufacturing products such as mineral oils, fats and/or waxes used previously which might interfere with the hydrophilic capabilities of the transmission layer. In some embodiments, an additional manufacturing step can subsequently be carried in which the 3D spacer fabric is washed in a hydrophilic agent (such as, but not limited to, Feran Ice 30g/l available from the Rudolph Group). This process step helps ensure that the surface tension on the materials is so low that liquid such as water can enter the fabric as soon as it contacts the 3D knit fabric. This also aids in controlling the flow of the liquid insult component of any exudates.

A layer 221 of absorbent material is provided above the transmission layer 226. The absorbent material, which comprise a foam or non-woven natural or synthetic material, and which may optionally comprise a super-absorbent material, forms a reservoir for fluid, particularly liquid, removed from the wound. In some embodiments, the layer 10 may also aid in drawing fluids towards the backing layer 220.

The material of the absorbent layer 221 may also prevent liquid collected in the wound dressing 100 from flowing freely within the dressing, and preferably acts so as to contain any liquid collected within the dressing. The absorbent layer 221 also helps distribute fluid throughout the layer via a wicking action so that fluid is drawn from the wound and stored throughout the absorbent layer. This helps prevent agglomeration in areas of the absorbent layer. The capacity of the absorbent material must be sufficient to manage the exudates flow rate of a wound when negative pressure is applied. Since in use the absorbent layer experiences negative pressures the material of the absorbent layer is chosen to absorb liquid under such circumstances. A number of materials exist that are able to absorb liquid when under negative pressure, for example superabsorber material. The absorbent layer 221 may typically be manufactured from ALLEVYN^{™} foam, Freudenberg 114-224-4 and/or Chem-Posite^{™}11C-450. In some embodiments, the absorbent layer 221 may comprise a composite comprising superabsorbent powder, fibrous material such as cellulose, and bonding fibers. In a preferred embodiment, the composite is an airlaid, thermally-bonded composite.

In some embodiments, the absorbent layer 221 is a layer of non-woven cellulose fibers having super-absorbent material in the form of dry particles dispersed throughout. Use of the cellulose fibers introduces fast wicking elements which help quickly and evenly distribute liquid taken up by the dressing. The juxtaposition of multiple strand-like fibers leads to strong capillary action in the fibrous pad which helps distribute liquid. In this way, the super-absorbent material is efficiently supplied with liquid. The wicking action also assists in bringing liquid into contact with the upper cover layer to aid increase transpiration rates of the dressing.

An aperture, hole, or orifice 227 is preferably provided in the backing layer 220 to allow a negative pressure to be applied to the dressing 100. The fluidic connector 110 is preferably attached or sealed to the top of the backing layer 220 over the orifice 227 made into the dressing 100, and communicates negative pressure through the orifice 227. A length of tubing may be coupled at a first end to the fluidic connector 110 and at a second end to a pump unit (not shown) to allow fluids to be pumped out of the dressing. Where the fluidic connector is adhered to the top layer of the wound dressing, a length of tubing may be coupled at a first end of the fluidic connector such that the tubing, or conduit, extends away from the fluidic connector parallel or substantially to the top surface of the dressing. The fluidic connector 110 may be adhered and sealed to the backing layer 220 using an adhesive such as an acrylic, cyanoacrylate, epoxy, UV curable or hot melt adhesive. The fluidic connector 110 may be formed from a soft polymer, for example a polyethylene, a polyvinyl chloride, a silicone or polyurethane having a hardness of 30 to 90 on the Shore A scale. In some embodiments, the fluidic connector 110 may be made from a soft or conformable material.

Preferably the absorbent layer 221 includes at least one through hole 228 located so as to underlie the fluidic connector 110. The through hole 228 may in some embodiments be the same size as the opening 227 in the backing layer, or may be bigger or smaller. As illustrated in Figure 2B a single through hole can be used to produce an opening underlying the fluidic connector 110. It will be appreciated that multiple openings could alternatively be utilized. Additionally should more than one port be utilized according to certain embodiments of the present disclosure one or multiple openings may be made in the absorbent layer and the obscuring layer in registration with each respective fluidic connector. Although not essential to certain embodiments of the present disclosure the use of through holes in the super-absorbent layer may provide a fluid flow pathway which remains unblocked in particular when the absorbent layer is near saturation.

The aperture or through-hole 228 is preferably provided in the absorbent layer 221 beneath the orifice 227 such that the orifice is connected directly to the transmission layer 226 as illustrated in Figure 2B. This allows the negative pressure applied to the fluidic connector 110 to be communicated to the transmission layer 226 without passing through the absorbent layer 221. This ensures that the negative pressure applied to the wound is not inhibited by the absorbent layer as it absorbs wound exudates. In other embodiments, no aperture may be provided in the absorbent layer 221, or alternatively a plurality of apertures underlying the orifice 227 may be provided. In further alternative embodiments, additional layers such as another transmission layer or an obscuring layer such as described in International Patent Publication WO2014020440 may be provided over the absorbent layer 221 and beneath the backing layer 220.

The backing layer 220 is preferably gas impermeable, but moisture vapor permeable, and can extend across the width of the wound dressing 100. The backing layer 220, which may for example be a polyurethane film (for example, Elastollan SP9109) having a pressure sensitive adhesive on one side, is impermeable to gas and this layer thus operates to cover the wound and to seal a wound cavity over which the wound dressing is placed. In this way an effective chamber is made between the backing layer 220 and a wound where a negative pressure can be established. The backing layer 220 is preferably sealed to the wound contact layer 222 in a border region around the circumference of the dressing, ensuring that no air is drawn in through the border area, for example via adhesive or welding techniques. The backing layer 220 protects the wound from external bacterial contamination (bacterial barrier) and allows liquid from wound exudates to be transferred through the layer and evaporated from the film outer surface. The backing layer 220 preferably comprises two layers; a polyurethane film and an adhesive pattern spread onto the film. The polyurethane film is preferably moisture vapor permeable and may be manufactured from a material that has an increased water transmission rate when wet. In some embodiments the moisture vapor permeability of the backing layer increases when the backing layer becomes wet. The moisture vapor permeability of the wet backing layer may be up to about ten times more than the moisture vapor permeability of the dry backing layer.

The absorbent layer 221 may be of a greater area than the transmission layer 226, such that the absorbent layer overlaps the edges of the transmission layer 226, thereby ensuring that the transmission layer does not contact the backing layer 220. This provides an outer channel of the absorbent layer 221 that is in direct contact with the wound contact layer 222, which aids more rapid absorption of exudates to the absorbent layer. Furthermore, this outer channel ensures that no liquid is able to pool around the circumference of the wound cavity, which may otherwise seep through the seal around the perimeter of the dressing leading to the formation of leaks. As illustrated in Figures 2A-2B, the absorbent layer 221 may define a smaller perimeter than that of the backing layer 220, such that a boundary or border region is defined between the edge of the absorbent layer 221 and the edge of the backing layer 220.

As shown in Figure 2B, one embodiment of the wound dressing 100 comprises an aperture 228 in the absorbent layer 221 situated underneath the fluidic connector 110. In use, for example when negative pressure is applied to the dressing 100, a wound facing portion of the fluidic connector may thus come into contact with the transmission layer 226, which can thus aid in transmitting negative pressure to the wound even when the absorbent layer 221 is filled with wound fluids. Some embodiments may have the backing layer 220 be at least partly adhered to the transmission layer 226. In some embodiments, the aperture 228 is at least 1-2 mm larger than the diameter of the wound facing portion of the fluidic connector 11, or the orifice 227.

In particular for embodiments with a single fluidic connector 110 and through hole, it may be preferable for the fluidic connector 110 and through hole to be located in an off-center position as illustrated in Figure 2A. Such a location may permit the dressing 100 to be positioned onto a patient such that the fluidic connector 110 is raised in relation to the remainder of the dressing 100. So positioned, the fluidic connector 110 and the filter 214 may be less likely to come into contact with wound fluids that could prematurely occlude the filter 214 so as to impair the transmission of negative pressure to the wound.

Turning now to the fluidic connector 110, preferred embodiments comprise a sealing surface 216, a bridge 211 (corresponding to bridge 120 in Figures 1A-1B) with a proximal end 130 and a distal end 140, and a filter 214. The sealing surface 216 preferably forms the applicator previously described that is sealed to the top surface of the wound dressing. In some embodiments a bottom layer of the fluidic connector 110 may comprise the sealing surface 216. The fluidic connector 110 may further comprise an upper surface vertically spaced from the sealing surface 216, which in some embodiments is defined by a separate upper layer of the fluidic connector. In other embodiments the upper surface and the lower surface may be formed from the same piece of material. In some embodiments the sealing surface 216 may comprise at least one aperture 229 therein to communicate with the wound dressing. In some embodiments the filter 214 may be positioned across the opening 229 in the sealing surface, and may span the entire opening 229. The sealing surface 216 may be configured for sealing the fluidic connector to the cover layer of the wound dressing, and may comprise an adhesive or weld. In some embodiments, the sealing surface 216 may be placed over an orifice in the cover layer with optional spacer elements 215 configured to create a gap between the filter 214 and the transmission layer 226. In other embodiments, the sealing surface 216 may be positioned over an orifice in the cover layer and an aperture in the absorbent layer 220, permitting the fluidic connector 110 to provide air flow through the transmission layer 226. In some embodiments, the bridge 211 may comprise a first fluid passage 212 in communication with a source of negative pressure, the first fluid passage 212 comprising a porous material, such as a 3D knitted material, which may be the same or different than the porous layer 226 described previously. The bridge 211 is preferably encapsulated by at least one flexible film layer 208, 210 having a proximal and distal end and configured to surround the first fluid passage 212, the distal end of the flexible film being connected the sealing surface 216. The filter 214 is configured to substantially prevent wound exudate from entering the bridge, and spacer elements 215 are configured to prevent the fluidic connector from contacting the transmission layer 226. These elements will be described in greater detail below.

Some embodiments may further comprise an optional second fluid passage positioned above the first fluid passage 212. For example, some embodiments may provide for an air leak may be disposed at the proximal end of the top layer that is configured to provide an air path into the first fluid passage 212 and dressing 100 similar to the suction adapter as described in U.S. Patent No 8,801,685.

Preferably, the fluid passage 212 is constructed from a compliant material that is flexible and that also permits fluid to pass through it if the spacer is kinked or folded over. Suitable materials for the fluid passage 212 include without limitation foams, including open-cell foams such as polyethylene or polyurethane foam, meshes, 3D knitted fabrics, non-woven materials, and fluid channels. In some embodiments, the fluid passage 212 may be constructed from materials similar to those described above in relation to the transmission layer 226. Advantageously, such materials used in the fluid passage 212 not only permit greater patient comfort, but may also provide greater kink resistance, such that the fluid passage 212 is still able to transfer fluid from the wound toward the source of negative pressure while being kinked or bent.

In some embodiments, the fluid passage 212 may be comprised of a wicking fabric, for example a knitted or woven spacer fabric (such as a knitted polyester 3D fabric, Baltex 7970^{®}, or Gehring 879^{®}) or a nonwoven fabric. These materials selected are preferably suited to channeling wound exudate away from the wound and for transmitting negative pressure and/or vented air to the wound, and may also confer a degree of kinking or occlusion resistance to the fluid passage 212. In some embodiments, the wicking fabric may have a three-dimensional structure, which in some cases may aid in wicking fluid or transmitting negative pressure. In certain embodiments, including wicking fabrics, these materials remain open and capable of communicating negative pressure to a wound area under the typical pressures used in negative pressure therapy, for example between 40 to 150 mmHg. In some embodiments, the wicking fabric may comprise several layers of material stacked or layered over each other, which may in some cases be useful in preventing the fluid passage 212 from collapsing under the application of negative pressure. In other embodiments, the wicking fabric used in the fluid passage 212 may be between 1.5 mm and 6 mm; more preferably, the wicking fabric may be between 3 mm and 6 mm thick, and may be comprised of either one or several individual layers of wicking fabric. In other embodiments, the fluid passage 212 may be between 1.2-3 mm thick, and preferably thicker than 1.5 mm. Some embodiments, for example a suction adapter used with a dressing which retains liquid such as wound exudate, may employ hydrophobic layers in the fluid passage 212, and only gases may travel through the fluid passage 212. Additionally, and as described previously, the materials used in the system are preferably conformable and soft, which may help to avoid pressure ulcers and other complications which may result from a wound treatment system being pressed against the skin of a patient.

Preferably, the filter element 214 is impermeable to liquids, but permeable to gases, and is provided to act as a liquid barrier and to ensure that no liquids are able to escape from the wound dressing 100. The filter element 214 may also function as a bacterial barrier. Typically the pore size is 0.2µm. Suitable materials for the filter material of the filter element 214 include 0.2 micron Gore^{™} expanded PTFE from the MMT range, PALL Versapore^{™} 200R, and Donaldson^{™} TX6628. Larger pore sizes can also be used but these may require a secondary filter layer to ensure full bioburden containment. As wound fluid contains lipids it is preferable, though not essential, to use an oleophobic filter membrane for example 1.0 micron MMT-332 prior to 0.2 micron MMT-323. This prevents the lipids from blocking the hydrophobic filter. The filter element can be attached or sealed to the port and/or the cover film over the orifice. For example, the filter element 214 may be molded into the fluidic connector 110, or may be adhered to one or both of the top of the cover layer and bottom of the suction adapter 110 using an adhesive such as, but not limited to, a UV cured adhesive.

It will be understood that other types of material could be used for the filter element 214. More generally a microporous membrane can be used which is a thin, flat sheet of polymeric material, this contains billions of microscopic pores. Depending upon the membrane chosen these pores can range in size from 0.01 to more than 10 micrometers. Microporous membranes are available in both hydrophilic (water filtering) and hydrophobic (water repellent) forms. In some embodiments of the invention, filter element 214 comprises a support layer and an acrylic co-polymer membrane formed on the support layer. Preferably the wound dressing 100 according to certain embodiments of the present invention uses microporous hydrophobic membranes (MHMs). Numerous polymers may be employed to form MHMs. For example, the MHMs may be formed from one or more of PTFE, polypropylene, PVDF and acrylic copolymer. All of these optional polymers can be treated in order to obtain specific surface characteristics that can be both hydrophobic and oleophobic. As such these will repel liquids with low surface tensions such as multi-vitamin infusions, lipids, surfactants, oils and organic solvents.

MHMs block liquids whilst allowing air to flow through the membranes. They are also highly efficient air filters eliminating potentially infectious aerosols and particles. A single piece of MHM is well known as an option to replace mechanical valves or vents. Incorporation of MHMs can thus reduce product assembly costs improving profits and costs/benefit ratio to a patient.

The filter element 214 may also include an odor absorbent material, for example activated charcoal, carbon fiber cloth or Vitec Carbotec-RT Q2003073 foam, or the like. For example, an odor absorbent material may form a layer of the filter element 214 or may be sandwiched between microporous hydrophobic membranes within the filter element. The filter element 214 thus enables gas to be exhausted through the orifice. Liquid, particulates and pathogens however are contained in the dressing.

The wound dressing 100 may comprise spacer elements 215 in conjunction with the fluidic connector 110 and the filter 214. With the addition of such spacer elements 215 the fluidic connector 110 and filter 214 may be supported out of direct contact with the absorbent layer 220 and/or the transmission layer 226. The absorbent layer 220 may also act as an additional spacer element to keep the filter 214 from contacting the transmission layer 226. Accordingly, with such a configuration contact of the filter 214 with the transmission layer 226 and wound fluids during use may thus be minimized.

Similar to the embodiments of wound dressings described above, some wound dressings comprise a perforated wound contact layer with silicone adhesive on the skin-contact face and acrylic adhesive on the reverse. Above this bordered layer sits a transmission layer or a 3D spacer fabric pad. Above the transmission layer, sits an absorbent layer. The absorbent layer can include a superabsorbent non-woven (NW) pad. The absorbent layer can over-border the transmission layer by approximately 5mm at the perimeter. The absorbent layer can have an aperture or through-hole toward one end. The aperture can be about 10 mm in diameter. Over the transmission layer and absorbent layer lies a backing layer. The backing layer can be a high moisture vapor transmission rate (MVTR) film, pattern coated with acrylic adhesive. The high MVTR film and wound contact layer encapsulate the transmission layer and absorbent layer, creating a perimeter border of approximately 20 mm. The backing layer can have a 10 mm aperture that overlies the aperture in the absorbent layer. Above the hole can be bonded a fluidic connector that comprises a liquid-impermeable, gas-permeable semi-permeable membrane (SPM) or filter that overlies the aforementioned apertures.

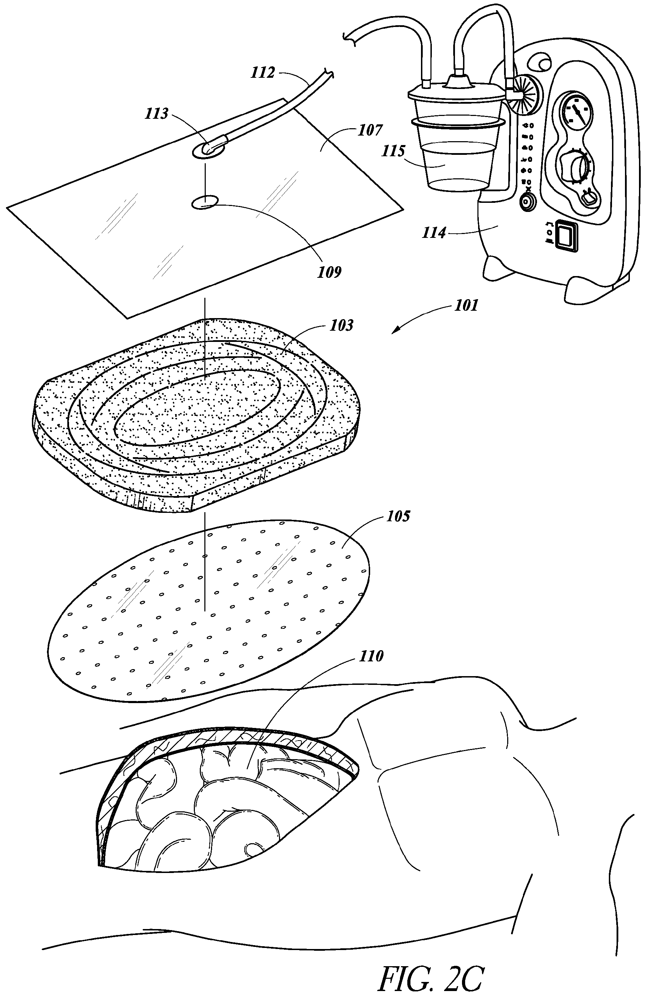

Turning to Figure 2C, treatment of other wound types, such as larger abdominal wounds, with negative pressure in certain embodiments uses a negative pressure treatment system 101 as illustrated schematically here. In this embodiment, a wound 106, illustrated here as an abdominal wound, may benefit from treatment with negative pressure. Such abdominal wounds may be a result of, for example, an accident or due to surgical intervention. In some cases, medical conditions such as abdominal compartment syndrome, abdominal hypertension, sepsis, or fluid edema may require decompression of the abdomen with a surgical incision through the abdominal wall to expose the peritoneal space, after which the opening may need to be maintained in an open, accessible state until the condition resolves. Other conditions may also necessitate that an opening-particularly in the abdominal cavity-remain open, for example if multiple surgical procedures are required (possibly incidental to trauma), or there is evidence of clinical conditions such as peritonitis or necrotizing fasciitis.

In cases where there is a wound, particularly in the abdomen, management of possible complications relating to the exposure of organs and the peritoneal space is desired, whether or not the wound is to remain open or if it will be closed. Therapy, preferably using the application of negative pressure, can be targeted to minimize the risk of infection, while promoting tissue viability and the removal of deleterious substances from the wound. The application of reduced or negative pressure to a wound has been found to generally promote faster healing, increased blood flow, decreased bacterial burden, increased rate of granulation tissue formation, to stimulate the proliferation of fibroblasts, stimulate the proliferation of endothelial cells, close chronic open wounds, inhibit burn penetration, and/or enhance flap and graft attachment, among other things. It has also been reported that wounds that have exhibited positive response to treatment by the application of negative pressure include infected open wounds, decubitus ulcers, dehisced incisions, partial thickness burns, and various lesions to which flaps or grafts have been attached. Consequently, the application of negative pressure to a wound106 can be beneficial to a patient.

Accordingly, certain embodiments provide for a wound contact layer 105 to be placed over the wound 106. The wound contact layer can also be referred to as an organ protection layer and/or a tissue protection layer. Preferably, the wound contact layer 105 can be a thin, flexible material which will not adhere to the wound or the exposed viscera in close proximity. For example, polymers such as polyurethane, polyethylene, polytetrafluoroethylene, or blends thereof may be used. In one embodiment, the wound contact layer is permeable. For example, the wound contact layer 105 can be provided with openings, such as holes, slits, or channels, to allow the removal of fluids from the wound 106 or the transmittal of negative pressure to the wound 106. Additional embodiments of the wound contact layer 105 are described in further detail below.

Certain embodiments of the negative pressure treatment system 101 may also use a porous wound filler 103, which can be disposed over the wound contact layer 105. This pad 103 can be constructed from a porous material, for example foam, that is soft, resiliently flexible, and generally conformable to the wound 106. Such a foam can include an open-celled and reticulated foam made, for example, of a polymer. Suitable foams include foams composed of, for example, polyurethane, silicone, and polyvinyl alcohol. Preferably, this pad 103 can channel wound exudate and other fluids through itself when negative pressure is applied to the wound. Some pads 103 may include preformed channels or openings for such purposes. In certain embodiments, the pad 103may have a thickness between about one inch and about two inches. The pad may also have a length of between about 16 and 17 inches, and a width of between about 11 and 12 inches. In other embodiments, the thickness, width, and/or length can have other suitable values. Other embodiments of wound fillers that may be used in place of or in addition to the pad 103 are discussed in further detail below.

Preferably, a drape 107 is used to seal the wound 106. The drape 107 can be at least partially liquid impermeable, such that at least a partial negative pressure may be maintained at the wound. Suitable materials for the drape 107 include, without limitation, synthetic polymeric materials that do not significantly absorb aqueous fluids, including polyolefins such as polyethylene and polypropylene, polyurethanes, polysiloxanes, polyamides, polyesters, and other copolymers and mixtures thereof. The materials used in the drape may be hydrophobic or hydrophilic. Examples of suitable materials include Transeal^{®} available from DeRoyal and OpSite^{®} available from Smith & Nephew. In order to aid patient comfort and avoid skin maceration, the drapes in certain embodiments are at least partly breathable, such that water vapor is able to pass through without remaining trapped under the dressing. An adhesive layer may be provided on at least a portion the underside of the drape 107 to secure the drape to the skin of the patient, although certain embodiments may instead use a separate adhesive or adhesive strip. Optionally, a release layer may be disposed over the adhesive layer to protect it prior to use and to facilitate handling the drape 107; in some embodiments, the release layer may be composed of multiple sections.

The negative pressure system 101 can be connected to a source of negative pressure, for example a pump 114. One example of a suitable pump is the Renasys EZ pump available from Smith & Nephew. The drape 107 may be connected to the source of negative pressure 114 via a conduit 112. The conduit 112 may be connected to a port 113 situated over an aperture 109 in the drape 107, or else the conduit 112 may be connected directly through the aperture 109 without the use of a port. In a further alternative, the conduit may pass underneath the drape and extend from a side of the drape. U.S. Pat. No. 7,524,315 discloses other similar aspects of negative pressure systems.

In many applications, a container or other storage unit 115 may be interposed between the source of negative pressure 114 and the conduit 112 so as to permit wound exudate and other fluids removed from the wound to be stored without entering the source of negative pressure. Certain types of negative pressure sources-for example, peristaltic pumps-may also permit a container 115 to be placed after the pump 114. Some embodiments may also use a filter to prevent fluids, aerosols, and other microbial contaminants from leaving the container 115 and/or entering the source of negative pressure 114. Further embodiments may also include a shut-off valve or occluding hydrophobic and/or oleophobic filter in the container to prevent overflow; other embodiments may include sensing means, such as capacitive sensors or other fluid level detectors that act to stop or shut off the source of negative pressure should the level of fluid in the container be nearing capacity. At the pump exhaust, it may also be preferable to provide an odor filter, such as an activated charcoal canister.

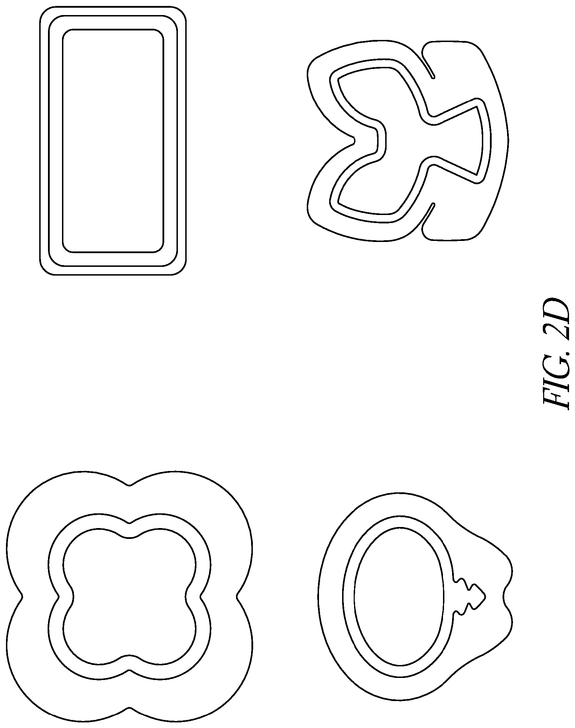

Figure 2D illustrates various embodiments of a wound dressing that can be used for healing a wound without negative pressure. As shown in the dressings of Figure 2D, the wound dressings can have multiple layers similar to the dressings described with reference to Figures 1A-1B and 2A-2B except the dressings of Figure 2D do not include a port or fluidic connector. The wound dressings of Figure 2D can include a cover layer and wound contact layer as described herein. The wound dressing can include various layers positioned between the wound contact layer and cover layer. For example, the dressing can include one or more absorbent layers and/or one or more transmission layers as described herein with reference to Figures 1A-1B and 2A-2B. Additionally, some embodiments related to wound treatment comprising a wound dressing described herein may also be used in combination or in addition to those described in U.S. Application Publication No. 2014/0249495, filed May 21, 2014, entitled "WOUND DRESSING AND METHOD OF TREATMENT" including further details relating to embodiments of wound dressings, the wound dressing components and principles, and the materials used for the wound dressings.

A wound dressing that incorporates a number of sensors can be utilized in order to monitor characteristics of a wound as it heals, provide therapy to the wound, monitor patient movement, etc. Collecting data from the wounds that heal well, and from those that do not, can provide useful insights towards identifying measurands to indicate whether a wound is on a healing trajectory.

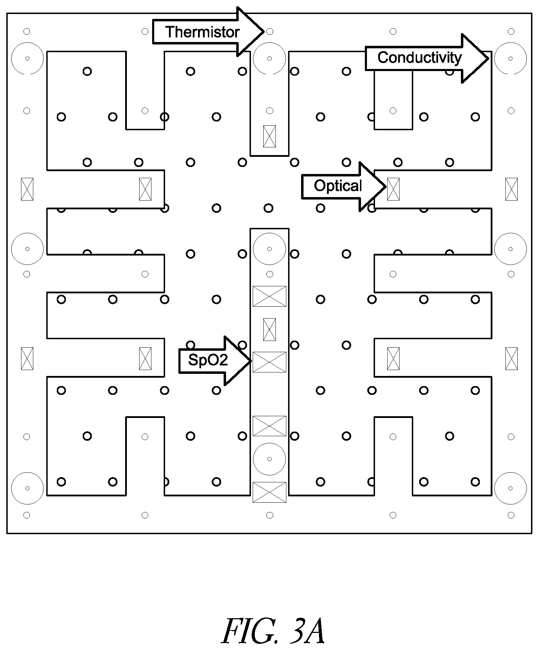

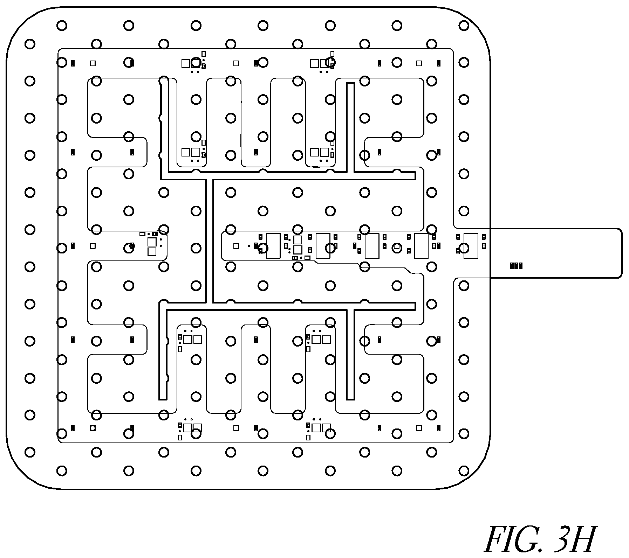

A number of sensor technologies can be used in wound dressings or one or more components forming part of an overall wound dressing apparatus. For example, as illustrated in Figure 3A and 3H, sub-sets of sensors can be incorporated onto or into a wound contact layer, which may be a perforated wound contact layer as shown in Figure 3H. The wound contact layer in Figure 3A and 3H is illustrated as having a square shape, but it will be appreciated that the wound contact layer may have other shapes such as rectangular, circular, oval, etc. In some embodiments, the sensor integrated wound contact layer can be provided as an individual material layer that is placed over the wound area and then covered by a wound dressing apparatus and/or components of a wound dressing apparatus similar to those described with reference to Figure 2C (e.g., gauze, foam or other wound packing material, a superabsorbent layer, a drape, a fully integrated dressing like the Pico or Allevyn Life dressing, etc.). In other embodiments, the sensor integrated wound contact layer may be part of a single unit dressing such as described in Figures 1A-2B and 2D.

The sensor integrated wound contact layer can be placed in contact with the wound and will allow fluid to pass through the contact layer while causing little to no damage to the tissue in the wound. The sensor integrated wound contact layer can be made of a flexible material such as silicone and can incorporate antimicrobials and/or other therapeutic agents known in the art. In some embodiments, the sensor integrated wound contact layer can incorporate adhesives that adhere to wet or dry tissue. In some embodiments, the sensors and/or sensor array can be incorporated into or encapsulated within other components of the wound dressing such as the absorbent layer and/or spacer layer described above.

As shown in Figures 3A and 3H, a sub-set of five sensors can be used including sensors for temperature (e.g., 25 thermistor sensors, in a 5 x 5 array, ~20mm pitch), SpO2 (e.g., 4 or 5 SpO2 sensors, in a single line from the center of the wound contact layer to the edge thereof, 10mm pitch), tissue color (e.g., 10 optical sensors, in 2 x 5 array, ~20mm pitch, electrical stimulation (e.g., electrodes), patient movement (e.g., an accelerometer, electromyography (EMG), magnetometer, gyroscope), pH (e.g., by measuring color of a pH-sensitive pad, optionally using the same optical sensors as for tissue color), and conductivity (e.g., 9 conductivity contacts, in a 3 x 3 array, ~40mm pitch). In some instances, more or fewer than five sensor can be utilized. Not all 5 sensors in each row of the array need be aligned. In some instances, all the sensors can be of the same type. In other instances, two or more different types of sensors can be used.

SpO2 is an estimate of arterial oxygen saturation. As shown in Figure 3A, the SpO2 sensors can be arranged in a single line from the center of or near the center of the wound contact layer to the edge of the wound contact layer. The line of SpO2 sensors can allow the sensor to take measurements in the middle of the wound, at the edge or the wound, and/or on intact skin to measure changes between the various regions. In some embodiments, the wound contact layer and/or sensor array can be larger than the size of the wound to cover the entire surface area of the wound as well as the surrounding intact skin. The larger size of the wound contact layer and/or sensor array and the multiple sensors can provide more information about the wound area than if the sensor was only placed in the center of the wound or in only one area at a time.

The sensors can be incorporated onto flexible circuit boards formed of flexible polymers including polyamide, polyimide (PI), polyester (PET), polyethylene naphthalate (PEN), polyetherimide (PEI), along with various fluropolymers (FEP) and copolymers, and/or any material known in the art. The sensor array can be incorporated into a two-layer flexible circuit. In some embodiments, the circuit board can be a multi-layer flexible circuit board. In some embodiments, these flexible circuits can be incorporated into any layer of the wound dressing. In some embodiments, a flexible circuit can be incorporated into a wound contact layer. For example, the flexible circuit can be incorporated into a wound contact layer similar to the wound contact layer described with reference to Figure 2B and 2C. The wound contact layer can have cutouts or slits that allow for one or more sensors to protrude out of the lower surface of the wound contact layer and contact the wound area directly.

In some embodiments, the sensor integrated wound contact layer can include a first and second wound contact layer with the flexible circuit board sandwiched between the two layers of wound contact layer material. The first wound contact layer has a lower surface intended to be in contact with the wound and an upper surface intended to be in contact with flexible circuit board. The second wound contact layer has a lower surface intended to be in contact with the flexible circuit board and an upper surface intended to be in contact with a wound dressings or one or more components forming part of an overall wound dressing apparatus. The upper surface of the first wound contact layer and the lower surface of the second wound contact layer can be adhered together with the flexible circuit board sandwiched between the two layers.

In some embodiments, the one or more sensors of the flexible circuit board can be fully encapsulated or covered by the wound contact layers to prevent contact with moisture or fluid in the wound. In some embodiments, the first wound contact layer can have cutouts or slits that allow for one or more sensors to protrude out of the lower surface and contact the wound area directly. For example, the one or more SpO2 sensors as shown in Figure 3H are shown protruding out the bottom surface of the wound contact layer. In some embodiments, the SpO2 sensors can be mounted directly on a lower surface of the first wound contact layer. Some or all of the sensors and electrical components may be potted or encapsulated (rendered waterproof) with a polymer, for example, silicon or epoxy based polymers. The encapsulation with a polymer can prevent ingress of fluid and leaching of chemicals from the components. In some embodiments, the wound contact layer material can seal the components from water ingress and leaching of chemicals.

The information gathered from the sensor array and associated wound dressing system can utilize three major components, including a sensor array, a control module, and software. These components are described in more detail below.

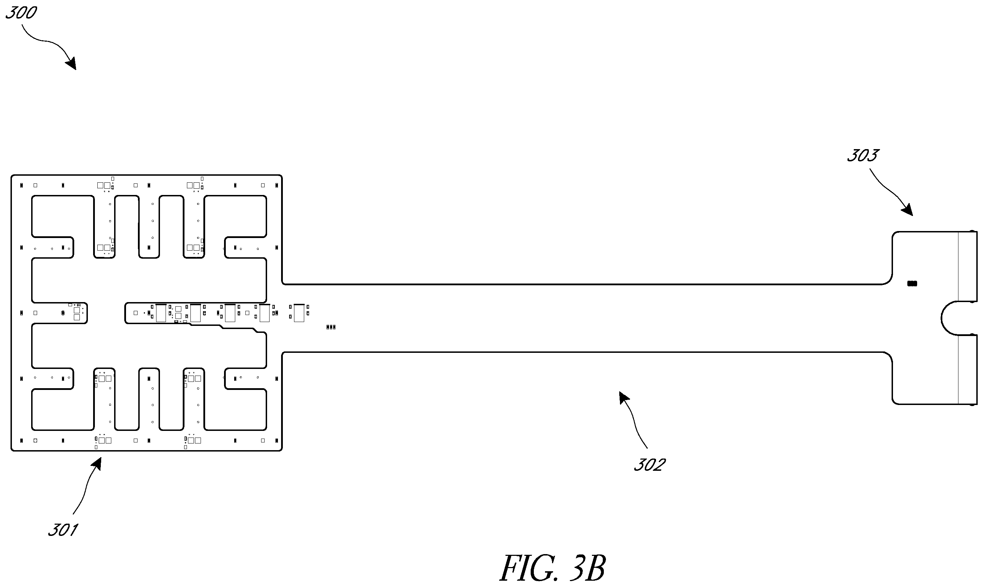

As described above, the sensor array of Figure 3A can include a thermistor, conductivity sensor, optical sensor, and SpO2 sensor. The flexible sensor array circuit board 300 includes a sensor array portion 301, a tail portion 302, and a connector pad end portion 303 as shown in Figure 3B. The sensor array portion 301 can include the sensors and associated circuitry. The sensor array circuit board 300 can include a long tail portion 302 extending from the sensor array portion 301. The connector pad end portion 303 can be enabled to connect to a control module or other processing unit to receive the data from the sensor array circuit. The long tail portion 302 can allow the control module to be placed distant from the wound and in a more convenient location. An overall view of one of the sensor arrays circuit board 300 is shown in Figure 3B.

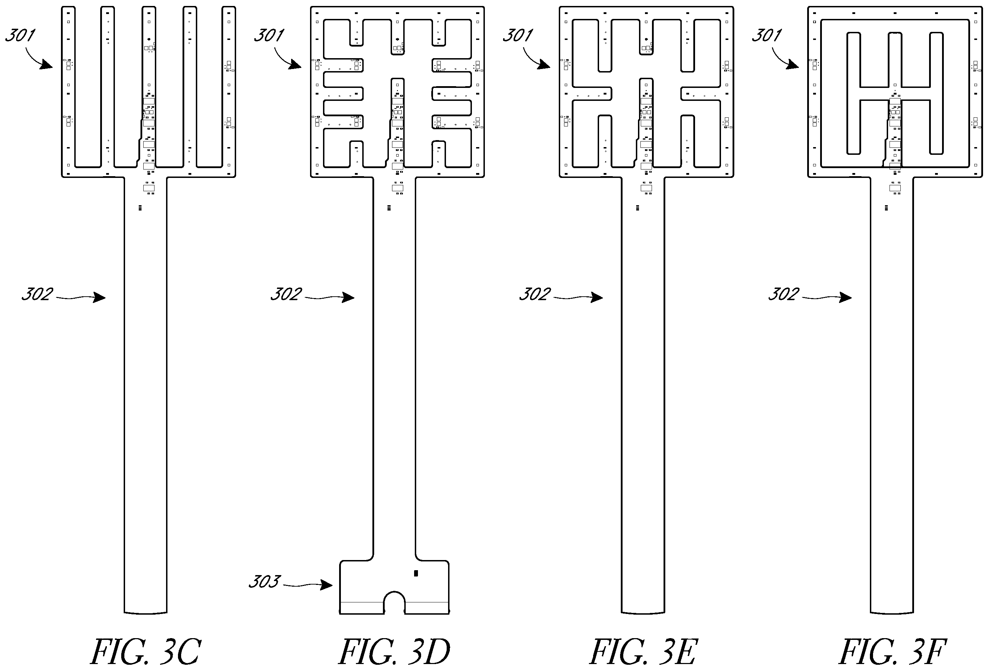

Figures 3C-3F show embodiments of the flexible circuit boards with four different sensor array geometries. The four different sensor array geometries shown are implemented in flexible circuits. While Figures 3C-3F show four different sensor array formats and configurations, the design as shown in Figure 3D also shows the connector pads end portion 303. However, the designs of Figures 3C, 3E, and 3F can also be created with the connector pads end portion 303 to allow these flexible circuit boards to communicate with a control module or other processing unit. Figure 3C-3F illustrate four different sensor array geometries in the sensor array portion 301.

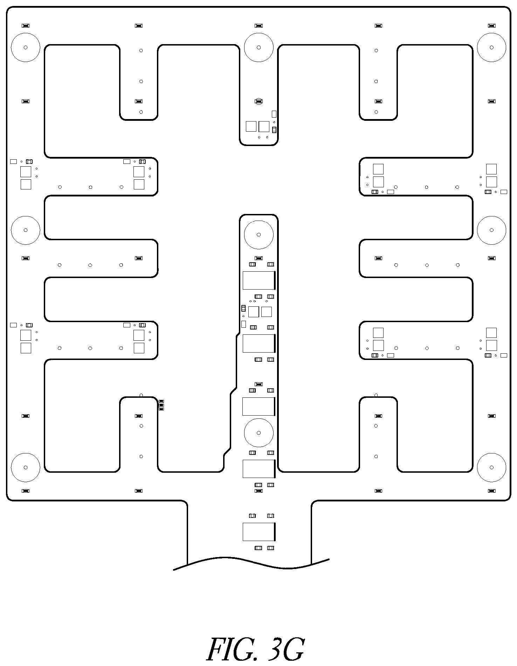

Figure 3G shows an embodiment of the sensor array portion 301 of the sensor array design shown in Figure 3D in more detail. In the embodiments of Figures3A-3G, it will be appreciated that the sensor array portion 301 includes a plurality of portions that extend either around a perimeter of a wound dressing component such as a wound contact layer, or inward from an outer edge of the wound dressing component. For example, the embodiments illustrated include a plurality of linearly extending portions that may be parallel to edges of a wound dressing component, and in some embodiments, follow the entire perimeter of the wound dressing component. In some embodiments, the sensor array portion may comprise a first plurality of parallel linearly extending portions that are perpendicular to a second plurality of parallel linearly extending portions. These linearly extending portions may also have different lengths and may extend inward to different locations within an interior of a wound dressing component. The sensor array portion preferably does not cover the entire wound dressing component, so that gaps are formed between portions of the sensor array. As shown in Figure 3A, this allows some, and possibly a majority of the wound dressing component to be uncovered by the sensor array. For example, for a perforated wound contact layer as shown in Figure 3A and 3H, the sensor array portion 301 may not block a majority of the perforations in the wound contact layer. In some embodiments, the sensor array may also be perforated or shaped to match the perforations in the wound contact layer to minimize the blocking of perforations to fluid flow.

Connectivity for the sensor array can vary depending on the various sensors and sensor array designs utilized. In some embodiments, as shown in Figure 3C-3F, a total of 79 connections can be used to connect the components of the sensor array. The sensor arrays can be terminated in two parallel 40-way 0.5mm pitch Flat Flexible Cable (FFC) contact surfaces, with terminals on the top surface, designed to be connected to an FFC connector such as Molex 54104-4031.

In some embodiments, thermistors, conductivity sensors, SpO2 sensors, and/or color sensors can be used on the sensor array to provide information relating to conditions of the wound. The sensor array and individual sensors can assist a clinician in monitoring the healing of the wound. The one or more sensors can operate individually or in coordination with each other to provide data relating to the wound and wound healing characteristics.

Temperature sensors can use thermocouples and/or thermistors to measure temperature. The thermistors can be used to measure and/or track the temperature of the underlying wound and/or the thermal environment within the wound dressing. The thermometry sensors can be calibrated and the data obtained from the sensors can be processed to provide information about the wound environment. In some embodiments, an ambient sensor measuring ambient air temperature can also be used to assist in eliminating problems associated with environment temperature shifts.

Optical sensors can be used to measure wound appearance using an RGB sensor with an illumination source. In some embodiments, both the RGB sensor and the illumination source would be pressed up against the skin, such that light would penetrate into the tissue and take on the spectral features of the tissue itself.

In some embodiments, pH-changing pads can be used as a pH sensor. A spectrometer and a broadband white light source can be used to measure the spectral response of the pH-changing pad. The illumination and imaging can be provided on the surface of the wound dressing that is in contact with the wound and at the bottom surface, which is exposed to fluid. Alternatively, in some embodiments, the illumination and imaging source can be provided on the surface of the wound dressing opposite the bottom surface and away from fluid application or the top surface of the dressing.

In some embodiments, pulse oximetry SpO2 sensors can be used. To measure how oxygenated the blood is and the pulsatile blood flow can be observed. Pulse oximetry measurements work by taking a time resolved measurement of light absorption / transmission in tissue at two different optical wavelengths. When hemoglobin becomes oxygenated, its absorption spectrum changes with regards to non-oxygenated blood. By taking a measurement at two different wavelengths, one gains a ratio metric measure of how oxygenated the blood is.

The components in the sensor array can be connected through multiple connections. In some embodiments, the thermistors can be arranged in groups of five. Each thermistor is nominally 10kΩ, and each group of five has a common ground. There are five groups of thermistors, giving a total of 30 connections. In some embodiments, there can be nine conductivity terminals. Each conductivity terminal requires one connection, giving a total of 9 connections. In some embodiments, there can be five SpO2 sensors. Each SpO2 sensor requires three connections, plus power and ground (these are covered separately), giving a total of 15 connections. In some embodiments, there can be 10 color sensors. Each color sensor comprises an RGB LED and an RGB photodiode. Each color sensor requires six connections, however five of these are common to every sensor, giving a total of 15 connections. Power and ground are considered separately. In some embodiments, there can be 5 pH sensors. The pH sensors can be a color-change discs, and can be sensed using the color sensors described above. Therefore, the pH sensors require no additional connections. There can be three power rails, and seven ground return signals, giving a total of 10 common connections. In some embodiments, the sensor array can include 25 thermistor (Murata NCP15WB473E03RC), 9 conductivity terminal, 5 SpO2 (ADPD144RI), 10 RGB LED (e.g. KPTF-1616RGBC-13), 10 RGB Color Sensor, 10 FET, a PCB, and an assembly.

Figure 3H illustrates a flexible sensor array incorporated into a perforated wound contact layer according to some embodiments. As shown in Figure 3H, the PCB sensor array can be sandwiched between two films or wound contact layers. The wound contact layers can have perforations formed as slits or holes as described herein that are small enough to help prevent tissue ingrowth into the wound dressing while allowing wound exudate to flow into the dressing. In some embodiments, the wound contact layers can have one or more slits that increase flexibility of the wound contact layer with integrated sensor array. In some embodiments, one of the wound contact layers can have extra cut outs to accommodate the sensors so that they can contact the skin directly.

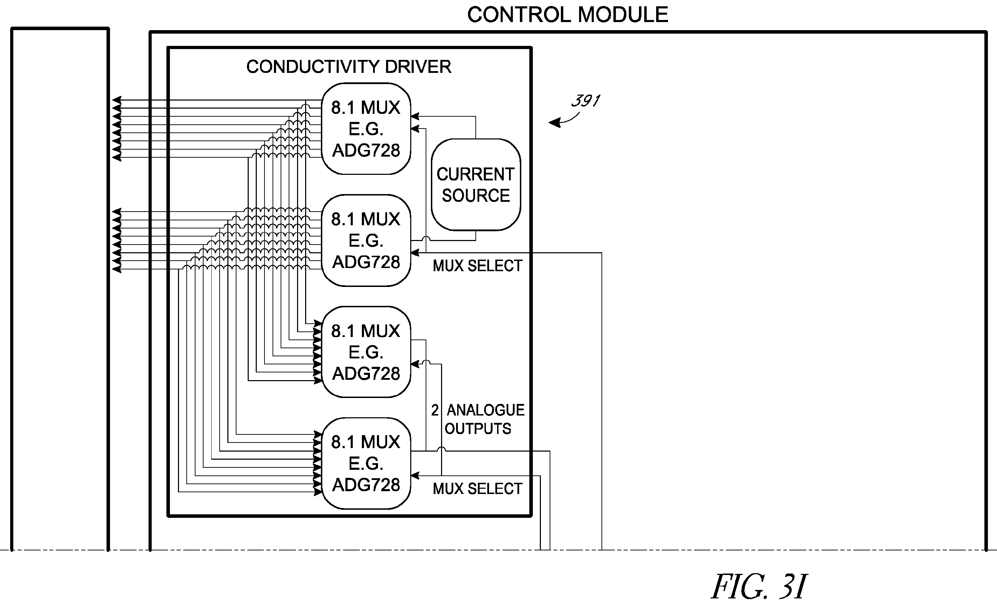

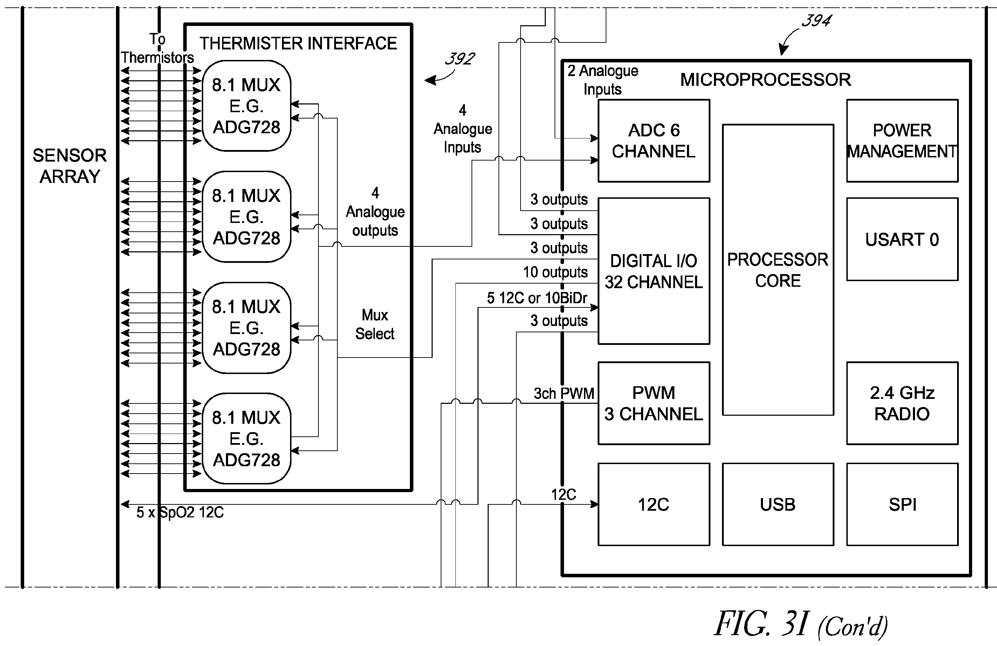

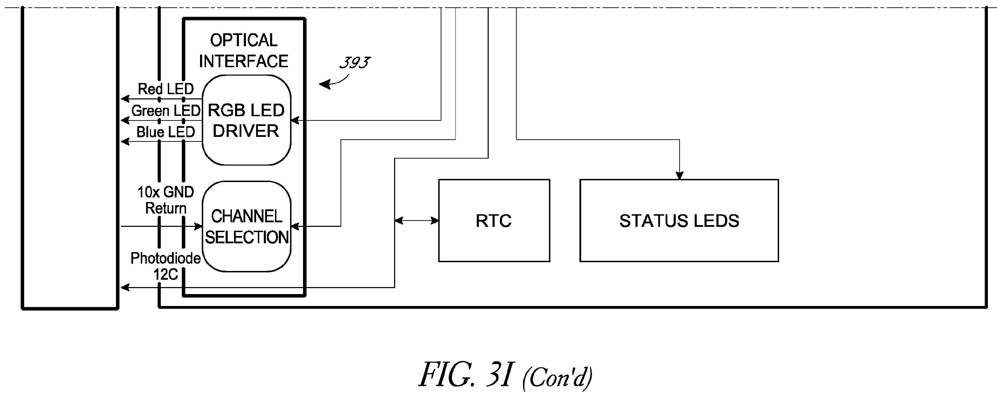

Figure 3I illustrates a block diagram of a control module according to some embodiments. The block diagram of the control module includes a conductivity driver box 391 displaying features of the conductivity driver. Box 392 shows the features of the thermistor interface and box 393 shows the features of the optical interface. The control module can include a microprocessor with features similar to those shown in box 394. Real time clock (RTC), Status LEDs, USB connector, Serial Flash, and Debug Connector can be included as features of the control module as shown in Figure 3I.

In some embodiments, the microprocessor can have one or more of the following requirements: 2.4GHz radio (either integrated, or external); Supplied Bluetooth software stack; SPI interface; USB (or UART for external USB driver); I2C; 3 channel PWM; 32 GPIO; and/or 6-channel ADC. In some embodiments, the device can require at least 48 I/O pins or possibly more due to banking limitations. Bluetooth stack typically requires ~20kB on-board Flash, so a minimum of 32kB can be required. In some embodiment, 64kB can be required if complex data processing is considered. The processor core can be ARM Cortex M4 or a similar processor core. In some embodiments, the parts can include ST's STM32L433LC or STM32F302R8, which would require an external radio, or NXP's Kinetis KW range including integrated radio.

In some embodiments, the control module can implement a memory component where the amount of local storage depends on the sample rate and resolution of the sensors. An estimated data requirement of 256Mb (32MB) is available in a serial Flash device from a number of manufacturers (Micron, Spansion).

The control module can utilize one or more analogue switches. In some embodiments, analogue switches with good on resistance and reasonable bandwidth can be used. For example, Analog Devices' ADG72 or NXP's NX3L4051HR can be used. Based on the initial system architecture, 8 of these will be required.

The control module can incorporate a battery. For example a 300mWh/day battery can be used. For 7 days this is 2100mWh. This could be provided by: a 10 days, non-rechargeable, ER14250 (14.5mm diameter x 25mm) LiSOCl2 cell; or a 7 days, rechargeable, Li 14500 (14.5mm diameter x 500mm) Li-Ion.

The control module can incorporate a real time clock (RTC). The RTC can be chosen from any RTC devices with crystal. The control module can also include miscellaneous resistors, capacitors, connectors, charge controllers, and other power supplies.

The PCB of the control module can be a 4-layer board, approximately 50mm x 20mm, or 25mm x 40mm. The type of PCB used can be largely driven by connection requirements to sensor array.

The enclosure of the control module can be a two part moulding, with clip features to allow easy access for changing sensor arrays or batteries.

The data collected through the sensor array can be passed through the control module and processed by a host software. The software may be executed on a processing device. The processing device can be a PC, tablet, smartphone, or other computer capable of running host software. The processing device executing the software can be in communication with the control module through electrical wires and/or through wireless communication. This software is not to perform the big-data analysis, but to provide access to the data held on the control module. Analysis software is beyond the scope of this document. The host software can include an interface to the control module via Bluetooth and/or USB. In some embodiments, the host software can read the status of control module, download logged data from control module, upload sample rate control to control module, convert data from control module into format suitable for processing by big-data analysis engine, and/or upload data to cloud for processing by analysis engine.

The software may be developed for PC (Windows / Linux), tablet or smartphone (Android / iOS), or for multiple platforms.

In some embodiments, a source of negative pressure (such as a pump) and some or all other components of the topical negative pressure system, such as power source(s), sensor(s), connector(s), user interface component(s) (such as button(s), switch(es), speaker(s), screen(s), etc.) and the like, can be integral with the wound dressing. In some embodiments, the components can be integrated below, within, on top of, and/or adjacent to the backing layer. In some embodiments, the wound dressing can include a second cover layer and/or a second filter layer for positioning over the layers of the wound dressing and any of the integrated components. The second cover layer can be the upper most layer of the dressing or can be a separate envelope that enclosed the integrated components of the topical negative pressure system.

As used herein the upper layer, top layer, or layer above refers to a layer furthest from the surface of the skin or wound while the dressing is in use and positioned over the wound. Accordingly, the lower surface, lower layer, bottom layer, or layer below refers to the layer that is closest to the surface of the skin or wound while the dressing is in use and positioned over the wound.

### Nanosensors

In some embodiments a wound dressing apparatus can incorporate or include one or more nanotechnology-enabled sensors (also referred to as nanosensors). The nanosensors can be utilized to measure changes in volume, concentration, displacement and velocity, gravitational, electrical, and magnetic forces, pressure, or temperature of cells in a body. Nanosensors may be able to distinguish between or recognize certain cells at the molecular level in order to deliver medicine or monitor development to specific places in the body. Nanosensors can detect characteristics of the wound which can be used to, for instance, monitor a wound and recommend a treatment plan based on how well it is healing. A set of nanosensors can work as a collective community. For example the nanosensors can communicate as a network and can be formulated into substrates (for example, foams or wound fillers which can be placed into a wound cavity).

As described herein with respect to other sensors, nanosensors can be incorporated into an array, a string, a flexible circuit board, a matrix, a chip, etc. In some embodiments, the nanosensors can be electronically printed on, for instance, a thin, light, disposable or flexible material. In some embodiments, the nanosensors are biocompatible.

As a wound heals, it can create electric fields. In some embodiments, the nanosensors can interpret and analyze the electrical signals given off by a wound. Thus, nanosensors can detect or precisely measure of those fields over time, thereby non-invasively tracking a healing process of a wound. In some embodiments, the nanosensors can track how fast or how well a wound is healing. In some embodiments, the nanosensors can accelerate wound healing.

In some embodiments, the nanosensors can communicate (for instance using incorporated antennae) with one or more other sensors or other communication device, such as a remote controller. The nanosensor data can be wirelessly transmitted and analyzed.

### Sensor Placement

Accurate placement of a sensor or a sensor array can be important to effective treatment of a wound or to effective data gathering. For example, different locations in and around wound can have drastically different characteristics. Without knowing where a sensor is located (for example, relative to the wound, other sensors, the patient, etc.), measured data can be misleading or inaccurate, thereby making it difficult to provide effective treatment to a patient. Accordingly, in some embodiments, one or more techniques are utilized to assist in increasing the accuracy of the sensor data. For example, one or more techniques are provided for reducing the chances of imperfect or incorrect placement. In addition, one or more techniques are provided for increasing the accuracy of sensor data despite imperfect or incorrect placement. Similarly, one or more techniques are provided which do not require specific, precise placement of sensors to gather accurate information.

In some embodiments, the position or orientation of one or more sensor strings, sensor strips, sensor arrays, or sensor matrices (generally referred to as sensor package), wounds, wound dressings, wound fillers, wound dressing apparatuses, etc. is tracked or determined and may be utilized to limit orientation errors. For example, alignment or orientation considerations may be taken with respect to how a sensor package is placed in or onto the wound to ensure that when the sensor package is installed or replaced, its orientation in each case is known. This can be necessary to co-reference and cross-reference data. In addition, the position or orientation data can be utilized to assist in the placement (e.g., initial placement or subsequent adjustments) of a wound dressing or sensor package to lessen the likelihood of imperfect placement. In addition, sensor data or sensor functionality can be modified based on the position or orientation data in order to increase the accuracy of sensor data despite imperfect placement.

In some embodiments, a sensor package can be utilized to limit orientation errors. For example, it may prove difficult to place a single sensor in a desired location because, for instance, the sensor may be small or difficult to orient correctly. A sensor package, on the other hand, can be easier to orient because, for example, the increased size or potential for orientation markers, as described herein.

In some embodiments, sensors or sensor package can be incorporated into or encapsulated within a wound dressing or wound packing material. For example, the sensors may be stitched into or otherwise permanently or semi-permanently attached to gauze or durafibre or one or more layers of the wound dressing. As another example, the sensors may be mounted onto foam protrusions which fit into wound. Still, in another example, a sensor or sensor package may be deployed into an expandable matrix, foam or other material which fills the wound.

### pH Sensing

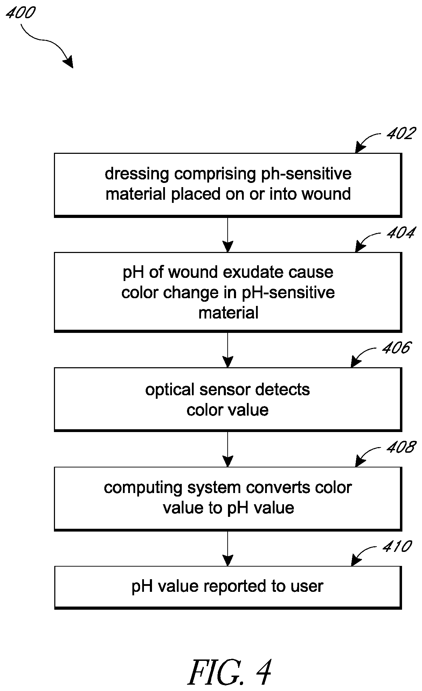

Figure 4 illustrates a wound treatment method 400 utilizing pH-sensitive material on a wound dressing according to some embodiments. In certain embodiments, a wound dressing or wound packing material comprising pH-sensitive materials may be placed on or in a wound 402. pH-sensitive materials may include: pH-sensitive dyes, pH-sensitive pigments, pH-sensitive inks, pH-sensitive superabsorbers, pH-sensitive adhesive or non-adhesive gels, pH-sensitive adhesive or non-adhesive foams, pH-sensitive hydrophilic polymers, pH-sensitive hydrophobic polymers, or other similar materials. For example, polyurethane gel matrices, such as those found in the CUTINOVA^{™} Hydro dressing by Smith & Nephew may be suitable as a pH-sensitive material with some modification. Such pH-sensitive materials incorporate an element (such as a dye molecule) that will change color at different pH values. As will be understood by one of skill in the art, the pH-sensitive element may be: attached directly to the backbone of the polymer via a chemical bond (such as ionic, covalent, and/or polar covalent), adsorbed to the polymer, adhered to the polymer, or attached via some other suitable means. As will also be understood by one of skill in the art, pH-sensing or pH-sensitive materials may be used synonymously throughout this specification.

In certain embodiments, the pH-sensitive element may be impregnated into specific types of polyurethanes having a higher hydrophilicity, for example EU33 (BASF Elastogram SP9109 polyurethane). Alternatively, more hydrophobic polyurethanes may be used, such as Kystsallgran PE399-100. For testing of tensile strength, polyurethanes may be extruded into dumbbell pieces within thicknesses ranging from about 1mm - 10 mm, or about 2mm.

Therefore, in some embodiments, a pH-sensitive material will become a particular color or provide a suitable indicator when exposed to wound exudate in block 404. In certain embodiments, a pH-sensitive element may be in the form of a triarylmethane dye, a fluorescent dye, or a phenylazo compound. The phenylazo compound may be in the form of: 2-[4(2-hydroxyethylsulfonyl)-phenyl]diazenyl]-4-methylphenol, 1-hydroxy-4-[4[(hydroxyethylsulphonyl)-phenylazo]-napthalene-2-sulphonate, 2-fluoro-4-[4[(2-hydroxyethanesulphonyl)-phenylazo]-6-methoxy phenol, 4-[4-(2-hydroxyethylsulphonyl)-phenylazo]-2,,6-dimethoxyphenol, or other suitable phenylazo compounds and/or combinations thereof. Further details regarding such pH-sensitive materials may be found in U.S. Pub. App. No. US2015/0308994A1, filed November 8, 2014 and entitled PH INDICATOR DEVICE AND FORMULATION.

As a non-limiting example, a sample preparation protocol was developed for the preparation of a pH-sensitive material such as disclosed herein this specification:
1. Weigh out 2-[4(2-hydroxyethylsulfonyl)-phenyl]diazenyl]-4-methylphenol (GJM 514) (32mg) and 4-[4-(2-hydroxyethylsulphonyl)-phenylazo]-2,,6-dimethoxyphenol (GJM 534) (18mg) (1:0.5), add to this 280µl of sulphuric acid (conc.) and leave to react for 30 minutes
2. In a 500ml volumetric flask have 446ml DI water, add to this the dye solution (after the 30 minutes)
3. To this add 4.0ml of sodium hydroxide (32% w/v solution, such as 3.2g of sodium hydroxide pellets in 10 ml of deionized water)
4. Then add 50ml sodium carbonate solution (2.36M), and make up to the 500ml mark with DI water
5. Place polyurethane samples EU33 (BASF Elastogran SP9109 polyurethane) into a beaker
6. Add the dye solution to the polyurethane samples and leave to react for 2 hours with stirring
7. Remove the dye solution and then wash with DI water (250ml) and leave with gentle agitation for a short period of time and then remove the wash solution
8. Repeat this wash step until no more dye appears in the water
9. Wash the sample one last time with DI water (250ml)
10. The samples will then be evaluated by exposing to an acid solution then a basic solution and noting a color change

In some embodiments, the pH-sensing element (also known as a dye) (and thus the pH-sensitive material itself) may become a reddish color when exposed to acidic conditions (pH 1-6) and become a blueish color when exposed to basic conditions (pH 8-14). Such a pH-sensing material would therefore change color dependent upon the surrounding medium, for example wound exudate 402. In certain embodiments, such pH-sensing elements change color along a gradient, such that a highly acidic condition will be a deeper red, while a highly basic condition may be a deeper blue. Correspondingly, a mildly acidic condition may be a lighter red, such as pink, while a mildly basic condition may be a lighter blue. Colors or red and blue dyes are merely representative, and such dyes may involve a variety of colors or shades, such as dark and light. In some embodiments, pH-sensitive materials may be optimized for a particular range of pHs, for example a range of about: 1-14, 2-12, 3-11, 4-10. 5-9. 6-8. or approximately a pH of 7. In certain embodiments, a pH-sensitive material may be tuned for sensing pH in a particular location or medium, for example within a wound. For example, a particular pH-sensing element may be optimized to provide high resolution within a narrow band of pH values associated with a wound. The pH of a wound may be indicative of the healing state of a wound, potentially indicating the presence of infection or other wound states.

In certain embodiments, as shown in block 406, a sensor such as described above and/or elsewhere in the specification, for example an optical sensor, may be positioned such that the optical sensor can detect optical changes in the pH-sensing material. The phrase "optical sensor" may comprise any optical sensor disclosed herein this section or elsewhere in the specification, with or without a combined light source, the light source comprising any light source described herein this section or elsewhere in the specification. As described herein, in some embodiments, a pH-sensing material may change color in response to changes in wound exudate pH, therefore such a color change may be detectable by the optical sensor in block 406. Such an optical sensor may then be configured to transmit such information to a controller or computing system which may convert such optical readings of color change into a corresponding pH value as illustrated in block 408 using a pH table. The controller or computing system may output (visually, audibly, tactilely, or the like) the pH value to a user as shown in block 410 or utilize the pH value in another suitable way. In some embodiments, the controller or computing system may provide further information to a user relating to the consequences of a particular wound pH, such as the presence of infection and/or impaired healing. In certain embodiments, such an optical sensor may continuously monitor the pH-sensitive material, thereby detecting changes in color over time and providing updated information to the user over time.

A pH-sensing material may comprise any material described herein this section or elsewhere in the specification, for example, hydrophilic polymers, foams, and/or gels, both adhesive and non-adhesive. Such foams may change color in response to interaction with a liquid (such as wound exudate) and/or a solid surface that has a particular pH. Such pH-sensitive foam would therefore provide an indication of the pH of the liquid and/or solid surface of interest, depending upon the color of the foam. In certain embodiments, pH-sensing foam may be created from a variety of techniques. For example, a fully-formed solid foam may be soaked in a liquid comprising a pH-sensitive element (such as a liquid dye), thereby coating the outer surface and potentially the porous interior of the foam with pH-sensitive elements. In the case of a foam with unconnected pores and inner channels, only the outer portion of the foam may be coated. In some embodiments, the pH-sensitive elements may be added directly to the raw foaming material before and/or during mixing. Therefore, after solidification into foam, the pH-sensing elements may be distributed throughout the foam. In certain embodiments, the pH-sensing elements may be distributed homogenously or heterogeneously throughout the foam. Distribution of pH-sensing elements throughout the foam may be controlled during mixing and formation of the foam, such that pH-sensing elements are distributed more heavily in areas of the foam that may come into contact with a liquid of interest, such as wound exudate. In certain embodiments, the foam may be chosen such that the foam has hydrophilic and/or hydrophobic properties. Advantageously, hydrophilic foam may absorb and direct wound exudates, thereby soaking up and sequestering wound exudate for measurement.

Similarly to the foams described herein, in some embodiments, gels, hydrophilic polymers, or other suitable materials described herein this section or elsewhere in the specification may be impregnated with pH-sensitive elements to thereby allow the gel or other material to change color upon interaction with a liquid such as wound exudate. As with foams, gels or other materials may be soaked in a pH-sensitive liquid such as a liquid dye, thereby coating the internal and external surfaces of the gel with pH-sensitive elements. Similarly, pH-sensitive elements can incorporated into adhesive gel using a variety of techniques. For example, the pH-sensitive elements can simply be mixed with the adhesive gel prior to solidification. As with the foams described herein, such a gel may be hydrophilic or hydrophobic; however, advantageously, hydrophilic gels may allow for greater sequestering of wound exudate and therefore better resolution.

As described herein, any optical sensor (such as those described herein this section or elsewhere in the specification) and optionally an illumination element may be used in combination with the pH-sensitive material to create a pH sensor. For example, a spectrometer and a broadband white light source may be utilized to measure the spectral response of the pH-sensitive material. The illumination and imaging can be provided on the surface of the wound dressing in contact with the wound, for example the bottom surface. However, in some embodiments the sensor or sensors may be positioned on the sides, top, or inside of the dressing. In some embodiments, the illumination and optical sensor may be provided on the top surface of the wound dressing opposite the bottom surface containing the pH-sensitive material and measure pH through the dressing. Measurement through the dressing may be particularly enhanced by making the dressing at least partially transparent.

In some examples not being part of the invention, the optical sensor(s) need not be incorporated into the wound dressing, instead a color change of the pH-sensitive material may be detected utilizing an external device such as a camera on a mobile device or smartphone, or other suitable device.

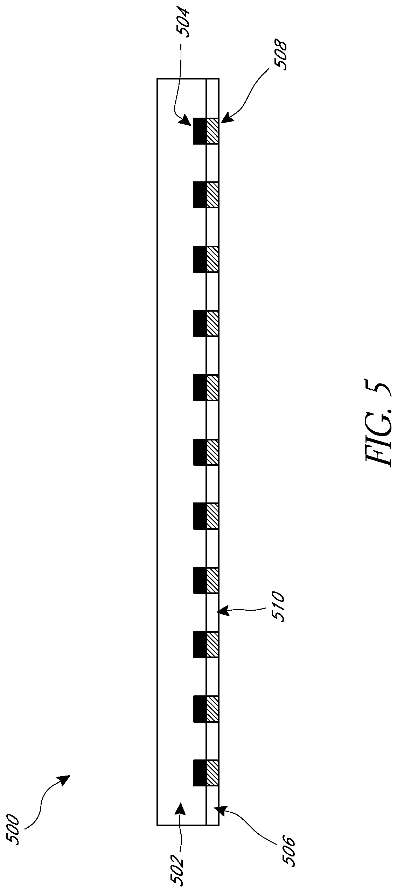

Figure 5 depicts a wound dressing 500, comprising optical sensors (with optional light sources) 504 and an adhesive layer 506, the adhesive layer containing pH-sensitive portions 508 beneath the optical sensor and non-pH sensitive portions 510, the non-pH sensitive portion(s) comprising materials such as any of those described herein this section and throughout the specification, except without pH-sensitive elements. For example, the non-pH sensitive portions may comprise hydrophilic adhesive gels or foams that do not contain pH-sensitive elements. In some embodiments, the pH-sensitive portion may comprise any pH-sensitive material described herein this section or elsewhere in the specification, for example, a pH-sensitive adhesive gel or foam. In some embodiments, such a pH-sensitive material may be transparent, partially transparent, or opaque. The wound dressing 500 may comprise but is not limited to any of the embodiments of wound dressings described above, and may or may not include any of the features of the other wound dressings described herein. The wound dressing 500 of Figure 5 may also be a component of a wound dressing, and may be a wound filler or a wound packing material. Figure 5 illustrates schematically a wound dressing layer 502 that may be a single layer of a wound dressing, or multiple layers as described in any of the embodiment above.

Such a pH-sensitive portion may be applied directly to the dressing such as to the dressing layer 502 via any suitable means, for example by printing or direct application with an extruder. Advantageously, a pH-sensitive adhesive gel or foam may be applied thickly on the dressing, thereby allowing for more sequestration of wound exudate and potentially greater resolution. A thicker adhesive layer allows for the absorption of more wound exudate, thereby allowing for a greater range of concentration of wound exudate in the range of the optical sensor. Due to the greater concentration of wound exudate in the thicker adhesive material, a greater color delta may be detected by the optical sensor, thereby providing an improved signal-to-noise ratio. As described herein, such an approach allows for any optical sensor positioned in a dressing to become a pH-detecting sensor, simply by coating the sensor in a layer of pH-sensitive material, such as a pH-sensitive gel or foam. The use of a gel or foam advantageously may improve resolution compared to a single thin surface layer of printed pH-sensitive dye overlying a portion of the dressing. Further, a pH-sensitive material impregnated with pH-sensing elements during forming will allow for the presence of color changing pH-sensitive elements throughout the pH-sensitive material, further enhancing resolution.

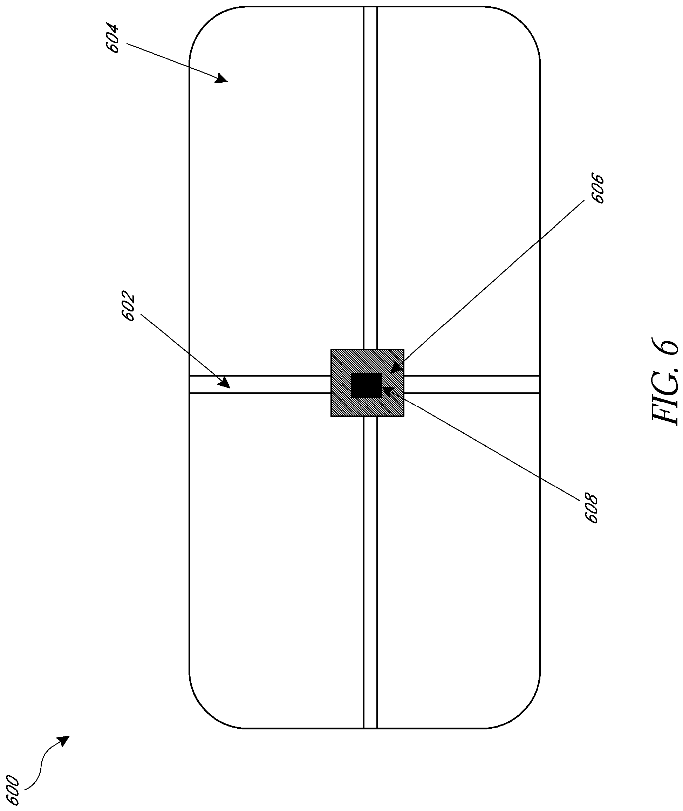

In certain embodiments and as depicted in the wound monitoring system 600 of Figure 6 (bottom view), wound exudate may be directed via channels 602 in a dressing 604 to pH-sensitive material 606 positioned under an optical sensor 608. In some embodiments, the pH-sensitive material may be positioned over an optical sensor. Similar to the non-pH sensitive portions depicted in Figure 5 above, the channels may comprise any material described herein this section or elsewhere in the specification, for example hydrophilic foam or gel adhesive. Since such channels may be hydrophilic, they may serve to direct wound exudate from other portions of the dressing to the pH-sensitive material over the optical sensor, thereby allowing the optical sensor to monitor the pH of wound exudate even when exudate is only coming into contact with distant portions of the dressing. The channels depicted in Figure 6 are only one possible position/orientation of the channels.

In some embodiments, the channels may encompass almost the entirety of a side of the dressing (interspersed with pH-sensitive materials/optical sensors), be positioned in a staggered pattern, a radial pattern, a spiral pattern, a matrix pattern or any suitable pattern to direct wound exudate to pH-sensitive material overlying one or more optical sensors.

### Sensor Normalization

A variety of factors may increase the variability of pH-sensor measurements over time and within different wounds. For example, variation in lighting, such as via different external light sources, changes in the color characteristics of the pH-sensitive material, changes in the pH-detecting sensor, and other aspects of the surroundings and the system itself may increase the variability of sensor measurements. Such variability may decrease the accuracy of color change measurements. Therefore, according to the invention, optical pH-detecting sensors such as those described above need to be normalized/calibrated to a reference material to avoid variability in optical sensor readings caused by factors other than the actual pH characteristics of the wound, such as described above. One of skill in the art will understand that normalization and calibration may be used interchangeably within this disclosure.

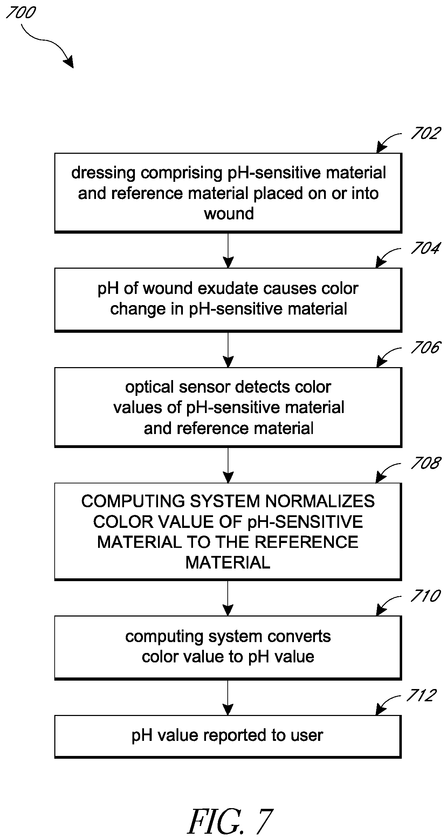

Figure 7 depicts an embodiment of a method for normalizing a pH-detecting sensor, such as the optical sensors described above in relation to Figures 4-6. According to the invention, a reference material with a known and fixed, stable color value is supplied with the optical sensor and wound dressing described above in relation to Figures 4-6. The reference material may be constructed from a non-absorbent material, such as a non-absorbent solid polymer. According to the invention, the reference material is incorporated in the dressing, such as in the top, bottom, or center of the dressing. The reference material is positioned within the dressing such that the optical sensor captures the reference material closely in space to the pH-sensitive material, thereby allowing one of skill to directly measure both the pH-sensitive material and the reference material at the same time and under the same lighting conditions. By comparing the pH-sensitive material to the reference material, changes in measurement due to changes in external lighting may be minimized.

Such reference material may be of any suitable material, provided the reference material is stable, with a long shelf life, and will not change in color or other optical characteristics over time. In embodiments, the reference material may be of any color, such as a color the corresponds to the color of the pH-sensitive material across a range of pH values corresponding to a wound. For example, the reference material may be within the color range of bluish to reddish, such as described above in relation to Figures 4-6.

Since the reference material will be of a stable color within different environments, comparison with the reference material will allow for increased accuracy of the optical sensor. Figure 7 illustrates a wound treatment method 700 for utilizing pH-sensitive material and a reference material in a wound dressing according to some embodiments. In certain embodiments, a wound dressing or wound filler material comprising pH-sensitive materials and a reference material may be placed on or in a wound 702. Such a dressing may be constructed in any suitable manner, such as described above in relation to Figures 4-6. For a reference material placed within a dressing, in embodiments the reference material may be positioned such that the color of the reference material does not change due to interaction with wound exudate. Further, as described above, the reference material is preferably non-absorbent, so as to not take up any wound exudate and potential alter the color.

Once the dressing or wound filler is placed in the wound, the pH-sensitive material may become a particular color or provide a suitable indicator when exposed to wound exudate in block 704. Similar to the method described above in relation to Figure 6, as shown in block 706, a sensor such as described above and/or elsewhere in the specification, for example an optical sensor, may be positioned such that the optical sensor can detect optical changes in the pH-sensing material. The phrase "optical sensor" may comprise any optical sensor disclosed herein this section or elsewhere in the specification, with or without a combined light source, the light source comprising any light source described herein this section or elsewhere in the specification.

As described above in relation to Figures 4-6, in some embodiments, a pH-sensing material may change color in response to changes in wound exudate pH, therefore such a color change is detectable by the optical sensor in block 706. Additionally, the optical sensor also collects color information from the reference material with a fixed known color value. As described elsewhere, the reference material is incorporated into the dressing close in space to the pH-sensitive material to account for lighting variation and other sources of variability. The optical sensor is constructed or positioned via any suitable means to collect optical data from both the pH-sensitive material and the reference material. For example, in an optical sensor with multiple sensor clusters, one sensor cluster may read the reference material and another sensor cluster may read the pH-sensitive material. Alternatively, a system could be set up with single LEDs and single optical sensors which then switch between measurements of the reference material and pH-sensitive material.

The optical sensor transmits such color information for the reference material and the pH-sensitive material to a controller or computing system. The controller of computing system normalizes 708 the pH-sensitive material to the reference material, because the reference material has a known, fixed color value. Therefore any deviation in color value for the reference material from the known value, is also used to normalize the color value of the pH-sensitive material.

Once the color value of the pH-sensitive material is normalized, the controller of computing system converts such optical color readings into a corresponding pH value as illustrated in block 710 using a pH table. The controller or computing system may output (visually, audibly, tactilely, or the like) the pH value to a user as shown in block 712 or utilize the pH value in a another suitable way. In some embodiments, the controller or computing system may provide further information to a user relating to the consequences of a particular wound pH, such as the presence of infection and/or impaired healing. As described above in relation to Figures 4-6, the measurements for both the pH-sensitive material and the reference material may be taken continuously over time, rather than as a snap-shot, such that changes in pH are monitored over time and automatically normalized.

### Other Variations

In some embodiments, one or more electronic components can be positioned on the side of a wound contact layer opposite the side that faces the wound. Systems and methods described herein are equally applicable to such wound contact layers. Any wound dressing embodiment described herein can include features of any of the other described wound dressing embodiments. Similarly, any controller described herein can include features of any of the other described wound dressing embodiments. Further, any device, component, or module described in a certain embodiment can include features of any of the other described embodiments of the device, component, or module.

Any value of a threshold, limit, duration, etc. provided herein is not intended to be absolute and, thereby, can be approximate. In addition, any threshold, limit, duration, etc. provided herein can be fixed or varied either automatically or by a user. Furthermore, as is used herein relative terminology such as exceeds, greater than, less than, etc. in relation to a reference value is intended to also encompass being equal to the reference value. For example, exceeding a reference value that is positive can encompass being equal to or greater than the reference value. In addition, as is used herein relative terminology such as exceeds, greater than, less than, etc. in relation to a reference value is intended to also encompass an inverse of the disclosed relationship, such as below, less than, greater than, etc. in relations to the reference value. Moreover, although blocks of the various processes may be described in terms of determining whether a value meets or does not meet a particular threshold, the blocks can be similarly understood, for example, in terms of a value (i) being below or above a threshold or (ii) satisfying or not satisfying a threshold.

Features, materials, characteristics, or groups described in conjunction with a particular aspect, embodiment, or example are to be understood to be applicable to any other aspect, embodiment or example described herein unless incompatible therewith. All of the features disclosed in this specification (including any accompanying claims, abstract and drawings), or all of the steps of any method or process so disclosed, may be combined in any combination, except combinations where at least some of such features or steps are mutually exclusive.

While certain embodiments have been described, these embodiments have been presented by way of example only, and are not intended to limit the scope of protection, which is solely defined by the appended claims. Indeed, the novel methods and systems described herein may be embodied in a variety of other forms. Furthermore, various omissions, substitutions and changes in the form of the methods and systems described herein may be made. Those skilled in the art will appreciate that in some embodiments, the actual steps taken in the processes illustrated or disclosed may differ from those shown in the figures. Depending on the embodiment, certain of the steps described above may be removed, others may be added. For example, the actual steps or order of steps taken in the disclosed processes may differ from those shown in the figure. Depending on the embodiment, certain of the steps described above may be removed, others may be added. For instance, the various components illustrated in the figures may be implemented as software or firmware on a processor, controller, ASIC, FPGA, or dedicated hardware. Hardware components, such as controllers, processors, ASICs, FPGAs, and the like, can include logic circuitry. Furthermore, the features and attributes of the specific embodiments disclosed above may be combined in different ways to form additional embodiments, all of which fall within the scope of the present disclosure.

The scope of the present invention is not intended to be limited by the specific disclosures of preferred embodiments herein, and is defined by claims as presented herein

Conditional language, such as "can," "could," "might," or "may," unless specifically stated otherwise, or otherwise understood within the context as used, is generally intended to convey that certain embodiments include, while other embodiments do not include, certain features, elements, or steps. Thus, such conditional language is not generally intended to imply that features, elements, or steps are in any way required for one or more embodiments or that one or more embodiments necessarily include logic for deciding, with or without user input or prompting, whether these features, elements, or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Further, the term "each," as used herein, in addition to having its ordinary meaning, can mean any subset of a set of elements to which the term "each" is applied.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, or 0.1 degree.

The scope of the present invention is not intended to be limited by the specific disclosures of preferred embodiments in this section or elsewhere in this specification, and is solely defined by claims. The language of the claims is to be interpreted broadly based on the language employed in the claims and not limited to the examples described in the present specification or during the prosecution of the application, which examples are to be construed as non-exclusive.

## Claims

1. A wound monitoring system, comprising:
a wound dressing (500, 604) configured to be positioned in contact with a wound, the wound dressing (500, 604) comprising an optical sensor (504, 608) configured to measure a color;
and
a pH-sensitive material (508, 606) positioned on an underside of the wound dressing (500, 604), the pH-sensitive material (508, 606) configured to change color in response to a change in a pH of the wound;
the system further comprising a reference material incorporated into the wound dressing (500, 604) closely in space to the pH-sensitive material, the reference material configured to maintain a stable, known and fixed color value which does not change over time, and
a controller or computing system,
wherein the optical sensor (504, 608) is configured to detect color information of the pH-sensitive material (508, 606) and of the reference material at the same time and under the same lighting conditions and to transmit the color information for the reference material and the pH-sensitive material to the controller or computing system, and
wherein the controller or computing system is configured to normalize the detected color information of the pH-sensitive material (508, 606) to the color information of the reference material, whereby any deviation in color value of the reference material from the known color value is used to normalize the color value of the pH-sensitive material, and to convert the normalized color information of the pH-sensitive material to a pH value.

2. The system of Claim 1, wherein the pH-sensitive material comprises a hydrophilic polymer, a gel, or a foam.

3. The system of any one of the preceding claims, wherein the pH-sensitive material is interspersed with pH-sensitive elements.

4. The system of Claim 3, wherein the pH-sensitive elements are dispersed heterogeneously.

5. The system of Claim 3, wherein the pH-sensitive elements are dispersed homogeneously.

6. The system of any of the preceding claims, wherein the pH-sensitive material comprises adhesive material.

7. The system of any of the preceding claims, wherein the pH-sensitive material comprises polyurethane.

8. The system of any of the preceding claims, further comprising a non-pH-sensitive material positioned on the underside of the wound dressing, the non-pH-sensitive material configured to direct wound exudate to the pH-sensitive material.

9. The system of Claim 8, wherein the non-pH-sensitive material comprises a hydrophilic polymer, a gel, or a foam.

10. The system of Claim 8 or 9, wherein the non-pH sensitive material is arranged as one or more channels on the underside of the wound dressing; optionally,
wherein the one or more channels extend from the pH-sensitive material to an edge of the dressing.

11. The system of any of Claims 8-10, wherein the non-pH-sensitive material comprises adhesive material.

12. The system of any of the preceding claims, wherein the controller is configured to provide an indication of the pH value to a user; optionally,
wherein the controller is further configured to display the pH value.

## Patentansprüche

1. Wundüberwachungssystem, umfassend:
einen Wundverband (500, 604), der dazu ausgelegt ist, in Kontakt mit einer Wunde positioniert zu werden, wobei der Wundverband (500, 604) einen optischen Sensor (504, 608) umfasst, der dazu ausgelegt ist, eine Farbe zu messen;
und
ein pH-empfindliches Material (508, 606), das auf einer Unterseite des Wundverbandes (500, 604) positioniert ist, wobei das pH-empfindliche Material (508, 606) dazu ausgelegt ist, in Reaktion auf eine Änderung eines pH-Werts der Wunde die Farbe zu ändern;
wobei das System ferner ein Referenzmaterial umfasst, das in den Wundverband (500, 604) in räumlich enger Nähe zu dem pH-empfindlichen Material eingebaut ist, wobei das Referenzmaterial dazu ausgelegt ist, einen stabilen, bekannten und festen Farbwert beizubehalten, der sich im Laufe der Zeit nicht ändert, und
eine Steuerung oder ein Rechensystem,
wobei der optische Sensor (504, 608) dazu ausgelegt ist, Farbinformationen des pH-empfindlichen Materials (508, 606) und des Referenzmaterials gleichzeitig und unter den gleichen Beleuchtungsbedingungen zu detektieren und die Farbinformationen für das Referenzmaterial und das pH-empfindliche Material an die Steuerung oder das Rechensystem zu übertragen, und
wobei die Steuerung oder das Rechensystem dazu ausgelegt ist, die detektierten Farbinformationen des pH-empfindlichen Materials (508, 606) auf die Farbinformationen des Referenzmaterials zu normalisieren, wobei eine beliebige Abweichung des Farbwerts des Referenzmaterials von dem bekannten Farbwert verwendet wird, um den Farbwert des pH-empfindlichen Materials zu normalisieren, und die normalisierten Farbinformationen des pH-empfindlichen Materials in einen pH-Wert umzuwandeln.

2. System nach Anspruch 1, wobei das pH-empfindliche Material ein hydrophiles Polymer, ein Gel oder einen Schaum umfasst.

3. System nach einem der vorhergehenden Ansprüche, wobei das pH-empfindliche Material mit pH-empfindlichen Elementen durchsetzt ist.

4. System nach Anspruch 3, wobei die pH-empfindlichen Elemente heterogen dispergiert sind.

5. System nach Anspruch 3, wobei die pH-empfindlichen Elemente homogen dispergiert sind.

6. System nach einem der vorhergehenden Ansprüche, wobei das pH-empfindliche Material Klebematerial umfasst.

7. System nach einem der vorhergehenden Ansprüche, wobei das pH-empfindliche Material Polyurethan umfasst.

8. System nach einem der vorhergehenden Ansprüche, ferner umfassend ein nicht-pH-empfindliches Material, das auf der Unterseite des Wundverbandes positioniert ist, wobei das nicht-pH-empfindliche Material dazu ausgelegt ist, Wundexsudat auf das pH-empfindliche Material zu leiten.

9. System nach Anspruch 8, wobei das nicht-pH-empfindliche Material ein hydrophiles Polymer, ein Gel oder einen Schaum umfasst.

10. System nach Anspruch 8 oder 9, wobei das nicht-pH-empfindliche Material als ein oder mehrere Kanäle auf der Unterseite des Wundverbandes angeordnet ist; wobei gegebenenfalls
sich der eine oder die mehreren Kanäle von dem pH-empfindlichen Material zu einem Rand des Verbands erstreckt/erstrecken.

11. System nach einem der Ansprüche 8-10, wobei das nicht-pH-empfindliche Material Klebematerial umfasst.

12. System nach einem der vorhergehenden Ansprüche, wobei die Steuerung dazu ausgelegt ist, einem Benutzer eine Angabe des pH-Werts bereitzustellen; wobei gegebenenfalls
die Steuerung ferner dazu ausgelegt ist, den pH-Wert anzuzeigen.

## Revendications

1. Système de surveillance de plaie, comprenant :
un pansement (500, 604) configuré pour être positionné au contact d'une plaie, le pansement (500, 604) comprenant un capteur optique (504, 608) configuré pour mesurer une couleur ;
et
un matériau sensible au pH (508, 606) positionné sur une face inférieure du pansement (500, 604), le matériau sensible au pH (508, 606) étant configuré pour changer de couleur en réponse à un changement d'un pH de la plaie ;
le système comprenant en outre un matériau de référence incorporé dans le pansement (500, 604) à proximité immédiate du matériau sensible au pH, le matériau de référence étant configuré pour maintenir une valeur de couleur stable, connue et fixe qui ne change pas au fil du temps, et
un contrôleur ou système informatique,
dans lequel le capteur optique (504, 608) est configuré pour détecter des informations de couleur du matériau sensible au pH (508, 606) et du matériau de référence en même temps et dans les mêmes conditions d'éclairage et pour transmettre les informations de couleur pour le matériau de référence et le matériau sensible au pH au contrôleur ou au système informatique, et
dans lequel le contrôleur ou le système informatique est configuré pour normaliser les informations de couleur détectées du matériau sensible au pH (508, 606) sur les informations de couleur du matériau de référence, tout écart de valeur de couleur du matériau de référence par rapport à la valeur de couleur connue étant utilisé pour normaliser la valeur de couleur du matériau sensible au pH, et pour convertir les informations de couleur normalisées du matériau sensible au pH en une valeur de pH.

2. Système selon la revendication 1, dans lequel le matériau sensible au pH comprend un polymère hydrophile, un gel ou une mousse.

3. Système selon l'une quelconque des revendications précédentes, dans lequel le matériau sensible au pH est parsemé d'éléments sensibles au pH.

4. Système selon la revendication 3, dans lequel les éléments sensibles au pH sont dispersés de manière hétérogène.

5. Système selon la revendication 3, dans lequel les éléments sensibles au pH sont dispersés de manière homogène.

6. Système selon l'une quelconque des revendications précédentes, dans lequel le matériau sensible au pH comprend un matériau adhésif.

7. Système selon l'une quelconque des revendications précédentes, dans lequel le matériau sensible au pH comprend du polyuréthane.

8. Système selon l'une quelconque des revendications précédentes, comprenant en outre un matériau non sensible au pH positionné sur la face inférieure du pansement, le matériau non sensible au pH étant configuré pour diriger un exsudat de la plaie vers le matériau sensible au pH.

9. Système selon la revendication 8, dans lequel le matériau non sensible au pH comprend un polymère hydrophile, un gel ou une mousse.

10. Système selon la revendication 8 ou 9, dans lequel le matériau non sensible au pH est agencé sous la forme d'un ou plusieurs canaux sur la face inférieure du pansement ; facultativement,
dans lequel le ou les canaux s'étendent à partir du matériau sensible au pH jusqu'à un bord du pansement.

11. Système selon l'une quelconque des revendications 8 à 10, dans lequel le matériau non sensible au pH comprend un matériau adhésif.

12. Système selon l'une quelconque des revendications précédentes, dans lequel le contrôleur est configuré pour fournir une indication de la valeur de pH à un utilisateur ; facultativement,
dans lequel le dispositif de commande est en outre configuré pour afficher la valeur de pH.
